# EUROPÄISCHE PATENTSCHRIFT

(11) **EP 0 929 548 B1**
(45) Veröffentlichungstag und Bekanntmachung des Hinweises auf die Patenterteilung: **26.06.2002**
(21) Anmeldenummer: 97944858.6
(22) Anmeldetag: 09.09.1997
(51) Int. Cl.: C07D 401/12, C07D 403/12, C07D 405/12, C07D 409/12, C07D 411/12, C07D 413/12, C07D 417/12, C07D 419/12, A01N 47/38

(54) **SUBSTITUIERTE CARBAMOYLTRIAZOLE UND IHRE VERWENDUNG ALS HERBIZIDE**
SUBSTITUTED CARBAMOYL TRIAZOLES AND THEIR USE AS HERBICIDES
CARBAMOYLTRIAZOLES SUBSTITUES ET LEUR UTILISATION COMME HERBICIDES

(30) Priorität: 19.09.1996 DE 19638245
(43) Veröffentlichungstag der Anmeldung: 21.07.1999
(73) Patentinhaber: BASF AKTIENGESELLSCHAFT, 67056 Ludwigshafen (DE)
(72) Erfinder: BAUMANN, Ernst, D-67373 Dudenhofen (DE); ZAGAR, Cyrill, D-67061 Ludwigshafen (DE); KARDORFF, Uwe, D-68159 Mannheim (DE); MISSLITZ, Ulf, D-67433 Neustadt (DE); WESTPHALEN, Karl-Otto, D-67346 Speyer (DE); WALTER, Helmut, D-67283 Obrigheim (DE); HAMPRECHT, Gerhard, D-69469 Weinheim (DE)
(86) Internationale Anmeldenummer: EP9704902
(87) Internationale Veröffentlichungsnummer: WO9812193

(56) Entgegenhaltungen:
- EP-A- 0 140 194
- EP-A- 0 225 175
- EP-A- 0 422 369
- US-A- 5 521 186
- P. RADDATZ ET AL.: "Renin inhibitors containing new P1-P1' dipeptide mimetics with heterocycles in P1' " JOURNAL OF MEDICINAL CHEMISTRY, Bd. 35, Nr. 19, 18.September 1992, WASHINGTON DC, US, Seiten 3525-36, XP002050635

## Beschreibung

Die vorliegende Erfindung betrifft neue substituierte Carbamoyltriazole der Formel I 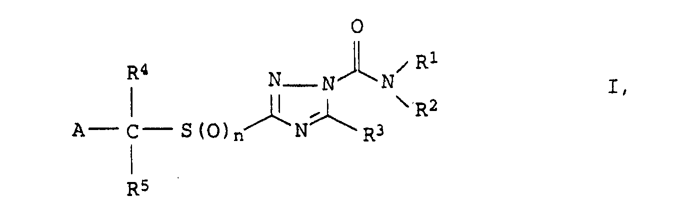 in der die Variablen folgende Bedeutung haben:
- R¹: Wasserstoff, C₁-C₆-Alkyl C₁-C₆-Elalogenalkyl, C₃-C₈-Cycloalkyl, C₃-C₆-Alkenyl oder C₃-C₆-Alkinyl;
- R²: C₁-C₆-Alkoxy oder C₁-C₆-Halogenalkoxy, sowie die unter R¹ genannten Reste;
oder
R¹ und R² bilden gemeinsam einen C₂-C₅-Alkandiyl-Rest, der ein bis drei Substituenten, ausgewählt aus der Gruppe Halogen und C₁-C₆-Alkyl, tragen kann, und im Fall eines C₄-C₅-Alkandiyl-Restes kann eine CH₂-Gruppe durch Sauerstoff oder eine Gruppe NH oder N-C₁-C₆-Alkyl ersetzt werden;
- R³,R⁴,R⁵: Wasserstoff, Cyano, C₁-C₆-Alkyl, C₁-C₆-Halogenalkyl, C₃-C₆-Cycloalkyl, C₂-C₆-Alkenyl, C₂-C₆-Alkinyl, C₁-C₆-Alkoxy, C₁-C₆-Halogenalkoxy, C₁-C₆-Alkylthio, C₁-C₆-Halogenalkylthio, C₁-C₆-Alkylcarbonyl, C₁-C₆-Halogenalkylcarbonyl, C₁-C₆-Alkoxycarbonyl, Phenyl oder Benzyl, wobei die beiden letztgenannten Substituenten partiell oder vollständig halogeniert sein können und eine bis drei der folgenden Gruppen tragen können: Nitro, Cyano, C₁-C₄-Alkyl, C₁-C₄-Halogenalkyl, C₁-C₄-Alkoxy, C₁-C₄-Halogenalkoxy und C₁-C₄-Alkoxycarbonyl;
- A: ggf. substituierter, gesättigter oder partiell ungesättigter Heterocyclus, der ein bis drei Heteroatome, ausgewählt aus der Gruppe Sauerstoff, Schwefel und Stickstoff enthält, und fünf- oder sechsgliedrig ist;
- n: 0, 1 oder 2;
mit der Maßgabe, daß A nicht für 1,3-Dioxolan-2-yl oder 1,3-Dioxan-2-yl steht;
sowie die landwirtschaftlich brauchbaren Salze von I.

Außerdem betrifft die Erfindung Verfahren zur Herstellung von Verbindungen der Formel I, Mittel, welche diese enthalten sowie deren Verwendung zur Schadpflanzenbekämpfung.

Aus der Literatur, beispielsweise aus EP-A 140 194, EP-A 422 369, US 5,308,830, JP-A 07 053 529 und JP-A 61 178 930 sind Carbamoyltriazole bekannt.

Die herbiziden Eigenschaften der bisher bekannten Verbindungen sowie die Verträglichkeiten gegenüber Kulturpflanzen können jedoch nur bedingt befriedigen. Es lag daher dieser Erfindung die Aufgabe zugrunde, neue, insbesondere herbizid wirksame Verbindungen mit verbesserten Eigenschaften zu finden.

Die Aufgabe erstreckt sich auch auf die Bereitstellung neuer Zwischenprodukte, den Triazolen der Formel II.

Demgemäß wurden die substituierten Carbamoyltriazole der Formel I sowie deren herbizide Wirkung gefunden. Ebenso wurden Zwischenprodukte und Verfahren zur Herstellung der Verbindungen I gefunden.

Ferner wurden herbizide Mittel gefunden, die die Verbindungen I enthalten und eine sehr gute herbizide Wirkung besitzen. Außerdem wurden Verfahren zur Herstellung dieser Mittel und Verfahren zur Bekämpfung von unerwünschtem Pflanzenwuchs mit den Verbindungen I gefunden.

Die Verbindungen der Formel I können je nach Substitutionsmuster ein oder mehrere Chiralitätszentren enthalten und liegen dann als Enantiomeren oder Diastereomerengemische vor. Gegenstand der Erfindung sind sowohl die reinen Enantiomeren oder Diastereomeren als auch deren Gemische.

Die Verbindungen der Formel I können auch in Form ihrer landwirtschaftlich brauchbaren Salze vorliegen, wobei es auf die Art des Salzes in der Regel nicht ankommt. Im allgemeinen kommen die Salze derjenigen Kationen oder die Säureadditionssalze derjenigen Säuren in Betracht, deren Kationen, beziehungsweise Anionen, die herbizide Wirkung der Verbindungen I nicht negativ beeinträchtigen. Es kommen also Kationen, insbesondere Ionen der Alkalimetalle, vorzugsweise Natrium und Kalium, der Erdalkalimetalle, vorzugsweise Calcium und Magnesium, und der Übergangsmetalle, vorzugsweise Mangan, Kupfer, Zink und Eisen, sowie Ammonium, das gewünschtenfalls ein bis vier C₁-C₄-Alkylsubstituenten und/oder einen Phenyl- oder Benzylsubstituenten tragen kann, vorzugsweise Diisopropylammonium, Tetramethylammonium, Tetrabutylammonium, Trimethylbenzylammonium, des weiteren Phosphoniumionen, Sulfoniumionen, vorzugsweise Tri(C₁-C₄-alkyl)sulfonium und Sulfoxoniumionen, vorzugsweise Tri(C₁-C₄-alkyl)sulfoxonium, in Betracht.
Anionen von brauchbaren Säureadditionssalzen sind in erster Linie Chlorid, Bromid, Fluorid, Hydrogensulfat, Sulfat, Dihydrogenphosphat, Hydrogenphosphat, Nitrat, Hydrogencarbonat, Carbonat, Hexafluorosilikat, Hexafluorophosphat, Benzoat sowie die Anionen von C₁-C₄-Alkansäuren, vorzugsweise Formiat, Acetat, Propionat und Butyrat.

Bevorzugt sind substituierte Carbamoyltriazole der Formel I 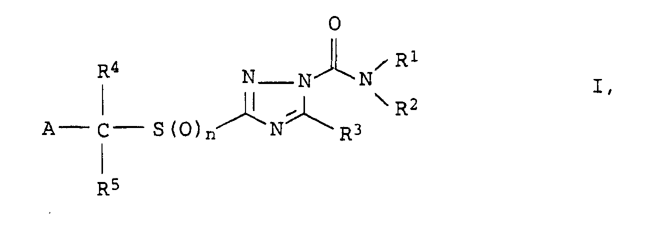 in der die Variablen folgende Bedeutung haben:
- R¹: Wasserstoff, C₁-C₆-Alkyl C₁-C₆-Halogenalkyl, C₃-C₈-Cycloalkyl, C₃-C₆-Alkenyl oder C₃-C₆-Alkinyl;
- R²: C₁-C₆-Alkoxy oder C₁-C₆-Halogenalkoxy, sowie die unter R¹ genannten Reste;
oder
R¹ und R² bilden gemeinsam einen C₂-C₅-Alkandiyl-Rest, der ein bis drei Substituenten, ausgewählt aus der Gruppe Halogen und C₁-C₆-Alkyl, tragen kann, und im Fall eines C₄-C₅-Alkandiyl-Restes kann eine CH₂-Gruppe durch Sauerstoff oder eine Gruppe NH oder N-C₁-C₆-Alkyl ersetzt werden;
- R³,R⁴,R⁵: Wasserstoff, Cyano, C₁-C₆-Alkyl, C₁-C₆-Halogenalkyl, C₃-C₆-Cycloalkyl, C₂-C₆-Alkenyl, C₂-C₆-Alkinyl, C₁-C₆-Alkoxy, C₁-C₆-Halogenalkoxy, C₁-C₆-Alkylthio, C₁-C₆-Halogenalkylthio, C₁-C₆-Alkylcarbonyl, C₁-C₆-Halogenalkylcarbonyl, C₁-C₆-Alkoxycarbonyl, Phenyl oder Benzyl, wobei die beiden letztgenannten Substituenten partiell oder vollständig halogeniert sein können und eine bis drei der folgenden Gruppen tragen können:
Nitro, Cyano, C₁-C₄-Alkyl, C₁-C₄-Halogenalkyl, C₁-C₄-Alkoxy, C₁-C₄-Halogenalkoxy und C₁-C₄-Alkoxycarbonyl;
- A: fünfgliedriger, gesättigter oder partiell ungesättigter Heterocyclus, der ein bis drei Heteroatome, ausgewählt aus der Gruppe Sauerstoff, Schwefel und Stickstoff, enthält und ein bis drei an Kohlenstoff gebundene Reste, ausgewählt aus der Gruppe R⁶, R⁷ und R⁸, tragen kann und wobei ggf. jeder Ringstickstoff unabhängig voneinander einen Rest R¹⁰ tragen kann;
oder
sechsgliedriger, gesättigter oder partiell ungesättigter Heterocyclus, der ein bis drei Heteroatome, ausgewählt aus der Gruppe Sauerstoff, Schwefel und Stickstoff, enthält und ein bis vier an Kohlenstoff gebundene Reste, ausgewählt aus der Gruppe R⁶, R⁷, R⁸ und R⁹, tragen kann und wobei ggf. jeder Ringstickstoff unabhängig voneinander einen Rest R¹⁰ tragen kann;
- R⁶,R⁷,R⁸,R⁹: Cyano, Halogen, C₁-C₆-Alkyl, C₁-C₆-Halogenalkyl, C₃-C₆-Cycloalkyl, C₂-C₆-Alkenyl, C₂-C₆-Alkinyl, C₁-C₆-Alkoxy, C₁-C₆-Halogenalkoxy, C₁-C₆-Alkylthio, C₁-C₆-Halogenalkylthio, C₁-C₆-Alkylcarbonyl, C₁-C₆-Halogenalkylcarbonyl, C₁-C₆-Alkoxycarbonyl, Phenyl oder Benzyl, wobei die beiden letztgenannten Substituenten partiell oder vollständig halogeniert sein können und/oder eine bis drei der folgenden Gruppen tragen können:
Nitro, Cyano, C₁-C₄-Alkyl, C₁-C₄-Halogenalkyl, C₁-C₄-Alkoxy, C₁-C₄-Halogenalkoxy und C₁-C₄-Alkoxycarbonyl;
- R¹⁰: C₁-C₆-Alkyl, C₁-C₆-Halogenalkyl, C₃-C₆-Cycloalkyl, C₃-C₆-Alkenyl, C₃-C₆-Alkinyl, C₁-C₆-Alkylcarbonyl, C₁-C₆-Halogenalkylcarbonyl, C₁-C₆-Alkoxycarbonyl, Phenyl oder Benzyl, wobei die beiden letztgenannten Substituenten partiell oder vollständig halogeniert sein können und/oder eine bis drei der folgenden Gruppen tragen können:
Nitro, Cyano, C₁-C₄-Alkyl, C₁-C₄-Halogenalkyl, C₁-C₄-Alkoxy, C₁-C₄-Halogenalkoxy und C₁-C₄-Alkoxycarbonyl;
- n: 0, 1 oder 2;
mit der Maßgabe, daß A nicht für 1,3-Dioxolan-2-yl oder 1,3-Dioxan-2-yl steht;
sowie die landwirtschaftlich brauchbaren Salze von I.

Die für die Substituenten R¹-R¹⁰ oder als Reste an Phenylringen genannten organischen Molekülteile stellen Sammelbegriffe für individuelle Aufzählungen der einzelnen Gruppenmitglieder dar. Sämtliche Kohlenwasserstoffketten, also alle Alkyl-, Halogenalkyl-, Alkoxy-, Halogenalkoxy-, Alkylthio-, Halogenalkylthio-, Alkylcarbonyl-, Halogenalkylcarbonyl-, Alkoxycarbonyl-, Cycloalkyl-, Alkandiyl-, Alkenyl-, Alkinyl-Teile können geradkettig oder verzweigt sein. Sofern nicht anders angegeben tragen halogenierte Substituenten vorzugsweise ein bis fünf gleiche oder verschiedene Halogenatome. Die Bedeutung Halogen steht jeweils für Fluor, Chlor, Brom oder Iod.

Ferner bedeuten beispielsweise:
- C₁-C₄-Alkyl sowie die Alkylteile von C₁-C₄-Alkylcarbonyl: Methyl, Ethyl, Propyl, 1-Methylethyl, Butyl, 1-Methylpropyl, 2-Methylpropyl und 1,1-Dimethylethyl;
- C₁-C₆-Alkyl, sowie die Alkylteile von C₁-C₆-Alkylcarbonyl: C₁-C₄-Alkyl, wie voranstehend genannt, sowie Pentyl, 1-Methylbutyl, 2-Methylbutyl, 3-Methylbutyl, 2,2-Dimethylpropyl, 1-Ethylpropyl, Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1-Ethyl-1-methylpropyl und l-Ethyl-3-methylpropyl;
- C₁-C₄-Halogenalkyl, sowie die Halogenalkylteile von C₁-C₄-Halogenalkylcarbonyl: einen C₁-C₄-Alkylrest wie vorstehend genannt, der partiell oder vollständig durch Fluor, Chlor, Brom und/oder Iod substituiert ist, also z.B. Chlormethyl, Dichlormethyl, Trichlormethyl, Fluormethyl, Difluormethyl, Trifluormethyl, Chlorfluormethyl, Dichlorfluormethyl, Chlordifluormethyl, 2-Fluorethyl, 2-Chlorethyl, 2-Bromethyl, 2-Iodethyl, 2,2-Difluorethyl, 2,2,2-Trifluorethyl, 2-Chlor-2-fluorethyl, 2-Chlor-2,2-difluorethyl, 2,2-Dichlor-2-fluorethyl, 2,2,2-Trichlorethyl, Pentafluorethyl, 2-Fluorpropyl, 3-Fluorpropyl, 2,2-Difluorpropyl, 2,3-Difluorpropyl, 2-Chlorpropyl, 3-Chlorpropyl, 2,3-Dichlorpropyl, 2-Brompropyl, 3-Brompropyl, 3,3,3-Trifluorpropyl, 3,3,3-Trichlorpropyl, 2,2,3,3,3-Pentafluorpropyl, Heptafluorpropyl, 1-(Fluormethyl)-2-fluorethyl, 1-(Chlormethyl)-2-chlorethyl, 1-(Brommethyl)-2-bromethyl, 4-Fluorbutyl, 4-Chlorbutyl, 4-Brombutyl und Nonafluorbutyl;
- C₁-C₆-Halogenalkyl, sowie die Halogenalkylteile von C₁-C₆-Halogenalkylcarbonyl: C₁-C₄-Halogenalkyl wie voranstehend genannt, sowie 5-Fluorpentyl, 5-Chlorpentyl, 5-Brompentyl, 5-Iodpentyl, Undecafluorpentyl, 6-Fluorhexyl, 6-Chlorhexyl, 6-Bromhexyl, 6-Iodhexyl und Dodecafluorhexyl;
- C₁-C₄-Alkoxy, sowie die Alkoxyteile in C₁-C₄-Alkoxycarbonyl: Methoxy, Ethoxy, Propoxy, 1-Methylethoxy, Butoxy, 1-Methylpropoxy, 2-Methylpropoxy und 1,1-Dimethylethoxy;
- C₁-C₆-Alkoxy, sowie die Alkoxyteile in C₁-C₆-Alkoxycarbonyl: C₁-C₄-Alkoxy wie voranstehend genannt, sowie Pentoxy, 1-Methylbutoxy, 2-Methylbutoxy, 3-Methoxylbutoxy, 1,1-Dimethylpropoxy, 1,2-Dimethylpropoxy, 2,2-Dimethylpropoxy, 1-Ethylpropoxy, Hexoxy, 1-Methylpentoxy, 2-Methylpentoxy, 3-Methylpentoxy, 4-Methylpentoxy, 1,1-Dimethylbutoxy, 1,2-Dimethylbutoxy, 1,3-Dimethylbutoxy, 2,2-Dimethylbutoxy, 2,3-Dimethylbutoxy, 3,3-Dimethylbutoxy, 1-Ethylbutoxy, 2-Ethylbutoxy, 1,1,2-Trimethylpropoxy, 1,2,2-Trimethylpropoxy, 1-Ethyl-1-methylpropoxy und 1-Ethyl-2-methylpropoxy;
- C₁-C₄-Halogenalkoxy: einen C₁-C₄-Alkoxyrest wie voranstehend genannt, der partiell oder vollständig durch Fluor, Chlor, Brom und/oder Iod substituiert ist, also z.B. Fluormethoxy, Difluormethoxy, Trifluormethoxy, Chlordifluormethoxy, Bromdifluormethoxy, 2-Fluorethoxy, 2-Chlorethoxy, 2-Bromethoxy, 2-Iodethoxy, 2,2-Difluorethoxy, 2,2,2-Trifluorethoxy, 2-Chlor-2-fluorethoxy, 2-Chlor-2,2-difluorethoxy, 2,2-Dichlor-2-fluorethoxy, 2,2,2-Trichlorethoxy, Pentafluorethoxy, 2-Fluorpropoxy, 3-Fluorpropoxy, 2-Chlorpropoxy, 3-Chlorpropoxy, 2-Brompropoxy, 3-Brompropoxy, 2,2-Difluorpropoxy, 2,3-Difluorpropoxy, 2,3-Dichlorpropoxy, 3,3,3-Trifluorpropoxy, 3,3,3-Trichlorpropoxy, 2,2,3,3,3-Pentafluorpropoxy, Heptafluorpropoxy, 1-(Fluormethyl)-2-fluorethoxy, 1-(Chlormethyl)-2-chlorethoxy, 1-(Brommethyl)-2-bromethoxy, 4-Fluorbutoxy, 4-Chlorbutoxy, 4-Brombutoxy und Nonafluorbutoxy;
- C₁-C₆-Halogenalkoxy: C₁-C₄-Halogenalkoxy wie voranstehend genannt, sowie 5-Fluorpentoxy, 5-Chlorpentoxy, 5-Brompentoxy, 5-Iodpentoxy, Undecafluorpentoxy, 6-Fluorhexoxy, 6-Chlorhexoxy, 6-Bromhexoxy, 6-Iodhexoxy und Dodecafluorhexoxy;
- C₁-C₆-Alkylthio: Methylthio, Ethylthio, Propylthio, 1-Methylethylthio, Butylthio, 1-Methylpropylthio, 2-Methylpropylthio, 1,1-Dimethylethylthio, Pentylthio, 1-Methylbutylthio, 2-Methylbutylthio, 3-Methylbutylthio, 2,2-Dimethylpropylthio, 1-Ethylpropylthio, Hexylthio, 1,1-Dimethylpropylthio, 1,2-Dimethylpropylthio, 1-Methylpentylthio, 2-Methylpentylthio, 3-Methylpentylthio, 4-Methylpentylthio, 1,1-Dimethylbutylthio, 1,2-Dimethylbutylthio, 1,3-Dimethylbutylthio, 2,2-Dimethylbutylthio, 2,3-Dimethylbutylthio, 3,3-Dimethylbutylthio, 1-Ethylbutylthio, 2-Ethylbutylthio, 1,1,2-Trimethylpropylthio, 1,2,2-Trimethylpropylthio, 1-Ethyl-1-methyl-propylthio und 1-Ethyl-2-methylpropylthio;
- C₁-C₆-Halogenalkylthio: einen C₁-C₆-Alkylthiorest wie voranstehend genannt, der partiell oder vollständig durch Fluor, Chlor, Brom und/oder Iod substituiert ist, also z.B. Fluormethylthio, Difluormethylthio, Trifluormethylthio, Chlordifluormethylthio, Bromdifluormethylthio, 2-Fluorethylthio, 2-Chlorethylthio, 2-Bromethylthio, 2-Iodethylthio, 2,2-Difluorethylthio, 2,2,2-Trifluorethylthio, 2,2,2-Trichlorethylthio, 2-Chlor-2-fluorethylthio, 2-Chlor-2,2-difluorethylthio, 2,2-Dichlor-2-fluorethylthio, Pentafluorethylthio, 2-Fluorpropylthio, 3-Fluorpropylthio, 2-Chlorpropylthio, 3-Chlorpropylthio, 2-Brompropylthio, 3-Brompropylthio, 2,2-Difluorpropylthio, 2,3-Difluorpropylthio, 2,3-Dichlorpropylthio, 3,3,3-Trifluorpropylthio, 3,3,3-Trichlorpropylthio, 2,2,3,3,3-Pentafluorpropylthio, Heptafluorpropylthio, 1-(Fluormethyl)-2-fluorethylthio, 1-(Chlormethyl)-2-chlorethylthio, 1-(Brommethyl)-2-bromethylthio, 4-Fluorbutylthio, 4-Chlorbutylthio, 4-Brombutylthio, Nonafluorbutylthio, 5-Fluorpentylthio, 5-Chlorpentylthio, 5-Brompentylthio, 5-Iodpentylthio, Undecafluorpentylthio, 6-Fluorhexylthio, 6-Chlorhexylthio, 6-Bromhexylthio, 6-Iodhexylthio und Dodecafluorhexylthio;
- C₂-C₅-Alkandiyl: Ethan-1,2-diyl, Propan-1,3-diyl, Butan-1,3-diyl und Pentan-1,5-diyl;
- C₃-C₆-Cycloalkyl: Cyclopropyl, Cyclobutyl, Cyclopentyl und Cyclohexyl;
- C₃-C₈-Cycloalkyl: einen C₃-C₆-Cycloalkylrest wie vorstehend genannt sowie Cycloheptyl und Cyclooctyl;
- C₃-C₆-Alkenyl: Prop-1-en-1-yl, Prop-2-en-1-yl, 1-Methylethenyl, Buten-1-yl, Buten-2-yl, Buten-3-yl, 1-Methyl-prop-1-en-1-yl, 2-Methyl-prop-1-en-1-yl, 1-Methyl-prop-2-en-1-yl, 2-Methyl-prop-2-en-1-yl, Penten-1-yl, Penten-2-yl, Penten-3-yl, Penten-4-yl, 1-Methyl-but-1-en-1-yl, 2-Methyl-but-1-en-1-yl, 3-Methyl-but-1-en-1-yl, 1-Methyl-but-2-en-1-yl, 2-Methyl-but-2-en-1-yl, 3-Methyl-but-2-en-1-yl, 1-Methyl-but-en-1-yl, 2-Methyl-but-3-en-1-yl, 3-Methyl-but-3-en-1-yl, 1,1-Dimethyl-prop-2-en-1-yl, 1,2-Dimethyl-prop-1-en-1-yl, 1,2-Dimethyl-prop-2-en-1-yl, 1-Ethyl-prop-1-en-2-yl, 1-Ethyl-prop-2-en-1-yl, Hex-1-en-1-yl, Hex-2-en-1-yl, Hex-3-en-1-yl, Hex-4-en-1-yl, Hex-5-en-1-yl, 1-Methyl-pent-l-en-1-yl, 2-Methyl-pent-1-en-1-yl, 3-Methyl-pent-1-en-1-yl, 4-Methyl-pent-1-en-1-yl, 1-Methyl-pent-2-en-1-yl, 2-Methyl-pent-2-en-1-yl, 3-Methyl-pent-2-en-1-yl, 4-Methyl-pent-2-en-1-yl, 1-Methyl-pent-3-en-1-yl, 2-Methyl-pent-3-en-1-yl, 3-Methyl-pent-3-en-1-yl, 4-Methyl-pent-3-en-1-yl, 1-Methyl-pent-4-en-1-yl, 2-Methyl-pent-4-en-1-yl, 3-Methyl-pent-4-en-1-yl, 4-Methyl-pent-4-en-1-yl, 1,1-Dimethyl-but-2-en-1-yl, 1,1-Dimethyl-but-3-en-1-yl, 1,2-Dimethyl-but-1-en-1-yl, 1,2-Dimethyl-but-2-en-1-yl, 1,2-Dimethyl-but-3-en-1-yl, 1,3-Dimethyl-but-1-en-1-yl, 1,3-Dimethyl-but-2-en-1-yl, 1,3-Dimethyl-but-3-en-1-yl, 2,2-Dimethyl-but-3-en-1-yl, 2,3-Dimethyl-but-1-en-l-yl, 2,3-Dimethyl-but-2-en-1-yl, 2,3-Dimethyl-but-3-en-1-yl, 3,3-Dimethyl-but-1-en-1-yl, 3,3-Dimethyl-but-2-en-1-yl, 1-Ethyl-but-1-en-1-yl, 1-Ethyl-but-2-en-1-yl, 1-Ethyl-but-3-en-1-yl, 2-Ethyl-but-1-en-1-yl, 2-Ethyl-but-2-en-1-yl, 2-Ethyl-but-3-en-1-yl, 1,1,2-Trimethyl-prop-2-en-1-yl, 1-Ethyl-1-methyl-prop-2-en-1-yl, 1-Ethyl-2-methyl-prop-1-en-1-yl und 1-Ethyl-2-methyl-prop-2-en-1-yl;
- C₂-C₆-Alkenyl: einen C₃-C₆-Alkenylrest wie voranstehend genannt, sowie Ethenyl;
- C₃-C₆-Alkinyl: Prop-1-in-1-yl, Prop-2-in-1-yl, But-1-in-1-yl, But-1-in-3-yl, But-1-in-4-yl, But-2-in-1-yl, Pent-1-in-1-yl, Pent-1-in-3-yl, Pent-1-in-4-yl, Pent-1-in-5-yl, Pent-2-in-1-yl, Pent-2-in-4-yl, Pent-2-in-5-yl, 3-Methyl-but-1-in-3-yl, 3-Methyl-but-1-in-4-yl, Hex-1-in-1-yl, Hex-1-in-3-yl, Hex-1-in-4-yl, Hex-1-in-5-yl, Hex-1-in-6-yl, Hex-2-in-1-yl, Hex-2-in-4-yl, Hex-2-in-5-yl, Hex-2-in-6-yl, Hex-3-in-1-yl, Hex-3-in-2-yl, 3-Methyl-pent-1-in-1-yl, 3-Methyl-pent-1-in-3-yl, 3-Methyl-pent-1-in-4-yl, 3-Methyl-pent-1-in-5-yl, 4-Methyl-pent-l-in-1-yl, 4-Methyl-pent-2-in-4-yl und 4-Methyl-pent-2-in-5-yl;
- C₂-C₆-Alkinyl: einen C₃-C₆-Alkinylrest wie voranstehend genannt, sowie Ethinyl;

Im Hinblick auf die biologische Wirkung sind substituierte Carbamoyltriazole der Formel I bevorzugt, in der die Substituenten folgende Bedeutung besitzen, und zwar für sich allein oder in Kombination:
- R¹: C₁-C₆-Alkyl, C₁-C₆-Halogenalkyl, C₃-C₈-Cycloalkyl, C₃-C₆-Alkenyl oder C₃-C₆-Alkinyl;
insbesondere bevorzugt C₁-C₄-Alkyl wie Methyl, Ethyl, Propyl oder Isopropyl, C₁-C₄-Halogenalkyl wie Fluormethyl, Difluormethyl oder 2,2,2-Trifluorethyl, C₃-C₆-Cycloalkyl wie Cyclopropyl, Cyclopentyl oder Cyclohexyl, C₃-C₄-Alkenyl wie 2-Propenyl, 2-Butenyl, 3-Butenyl oder 1-Methyl-2-propenyl oder C₃-C₄-Alkinyl wie 2-Propinyl;
außerordentlich bevorzugt Methyl, Ethyl, Propyl oder Isopropyl;
- R²: C₁-C₆-Alkyl, C₁-C₆-Halogenalkyl, C₃-C₈-Cycloalkyl, C₃-C₆-Alkenyl, C₃-C₆-Alkinyl, C₁-C₆-Alkoxy oder C₁-C₆-Halogenalkoxy;
insbesondere bevorzugt C₁-C₄-Alkyl wie Methyl, Ethyl, Propyl oder Isopropyl, C₁-C₄-Halogenalkyl wie Fluormethyl, Difluormethyl oder 2,2,2-Trifluorethyl, C₃-C₆-Cycloalkyl wie Cyclopropyl, Cyclopentyl oder Cyclohexyl, C₃-C₄-Alkenyl wie 2-Propenyl, 2-Butenyl, 3-Butenyl oder 1-Methyl-2-propenyl, C₃-C₄-Alkinyl wie 2-Propinyl, C₁-C₄-Alkoxy wie Methoxy oder Ethoxy oder C₁-C₄-Halogenalkoxy wie Difluormethoxy; außerordentlich bevorzugt Methyl, Ethyl, Propyl oder Isopropyl;
oder R¹ und R² bilden gemeinsam einen Ethan-1,2-diyl-, Propan-1,3-diyl-, Butan-1,4-diyl-, Pentan-1,5-diyl- oder 3-Oxa-pentan-1,5-diyl-Rest aus, der ein bis drei C₁-C₆-Alkyl-Substituenten tragen kann;
insbesondere bevorzugt Propan-1,3-diyl, Butan-1,4-diyl, Pentan-1,5-diyl oder 3-Oxa-pentan-1,5-diyl;
- R³: Wasserstoff, Cyano, C₁-C₆-Alkyl, C₁-C₆-Halogenalkyl, C₃-C₆-Cycloalkyl, C₂-C₆-Alkenyl, C₂-C₆-Alkinyl, C₁-C₆-Alkoxy, C₁-C₆-Halogenalkoxy, C₁-C₆-Alkylthio, C₁-C₆-Halogenalkylthio, C₁-C₆-Alkylcarbonyl, C₁-C₆-Halogenalkylcarbonyl, C₁-C₆-Alkoxycarbonyl, Phenyl oder Benzyl;
insbesondere bevorzugt Wasserstoff, Cyano, C₁-C₄-Alkyl wie Methyl oder Ethyl, C₁-C₄-Halogenalkyl wie Trifluormethyl, C₃-C₆-Cycloalkyl wie Cyclopropyl, C₃-C₄-Alkinyl wie Prop-1-in-3-yl, C₃-C₄-Alkenyl wie Prop-1-en-3-yl, C₁-C₄-Alkoxy wie Ethoxy, C₁-C₄-Halogenalkoxy wie Difluormethoxy, C₁-C₄-Alkylthio wie Methylthio, C₁-C₄-Alkylcarbonyl wie Methylcarbonyl, C₁-C₄-Alkoxycarbonyl wie Ethoxycarbonyl, Phenyl oder Benzyl;
- R⁴,R⁵: Wasserstoff, C₁-C₆-Alkyl, C₁-C₆-Halogenalkyl, C₃-C₆-Cycloalkyl, C₂-C₆-Alkenyl, C₂-C₆-Alkinyl, C₁-C₆-Alkoxy oder C₁-C₆-Halogenalkoxy;
insbesondere bevorzugt Wasserstoff, C₁-C₄-Alkyl wie Methyl oder Ethyl, C₁-C₄-Halogenalkyl wie Trifluormethyl, C₃-C₆-Cycloalkyl wie Cyclopropyl, C₃-C₄-Alkinyl wie Prop-1-in-3-yl, C₃-C₄-Alkenyl wie Prop-1-en-3-yl, C₁-C₄-Alkoxy wie Ethoxy oder C₃-C₄-Alkenyl wie Difluormethoxy;
- A: Tetrahydrofuran-2-yl, Tetrahydrofuran-3-yl, Tetrahydrothien-2-yl, Tetrahydrothien-3-yl, Tetrahydropyrrol-2-yl, Tetrahydropyrrol-3-yl, 2,3-Dihydrofuran-2-yl, 2,3-Dihydrofuran-3-yl, 2,5-Dihydrofuran-2-yl, 2,5-Dihydrofuran-3-yl, 4,5-Dihydrofuran-2-yl, 4,5-Dihydrofuran-3-yl, 2,3-Dihydrothien-2-yl, 2,3-Dihydrothien-3-yl, 2,5-Dihydrothien-2-yl, 2,5-Dihydrothien-3-yl, 4,5-Dihydrothien-2-yl, 4,5-Dihydrothien-3-yl, 2,3-Dihydro-1H-pyrrol-2-yl, 2,3-Dihydro-1H-pyrrol-3-yl, 2,5-Dihydro-1H-pyrrol-2-yl, 2,5-Dihydro-1H-pyrrol-3-yl, 4,5-Dihydro-1H-pyrrol-2-yl, 4,5-Dihydro-1H-pyrrol-3-yl, 3,4-Dihydro-2H-pyrrol-2-yl, 3,4-Dihydro-2H-pyrrol-3-yl, 3,4-Dihydro-5H-pyrrol-2-yl, 3,4-Dihydro-5H-pyrrol-3-yl, Tetrahydropyrazol-3-yl, Tetrahydropyrazol-4-yl, Tetrahydroisoxazol-3-yl, Tetrahydroisoxazol-4-yl, Tetrahydroisoxazol-5-yl, 1,2-Oxathiolan-3-yl, 1,2-Oxathiolan-4-yl, 1,2-Oxathiolan-5-yl, Tetrahydroisothiazol-3-yl, Tetrahydroisothiazol-4-yl, Tetrahydroisothiazol-5-yl, 1,2-Dithiolan-3-yl, 1,2-Dithiolan-4-yl, Tetrahydroimidazol-2-yl, Tetrahydroimidazol-4-yl, Tetrahydrooxazol-2-yl, Tetrahydrooxazol-4-yl, Tetrahydrooxazol-5-yl, Tetrahydrothiazol-2-yl, Tetrahydrothiazol-4-yl, Tetrahydrothiazol-5-yl, 1,3-Dioxolan-4-yl, 1,3-Oxathiolan-2-yl, 1,3-Oxathiolan-4-yl, 1,3-Oxathiolan-5-yl, 1,3-Dithiolan-2-yl, 1,3-Dithiolan-4-yl, 4,5-Dihydro-1H-pyrazol-3-yl, 4,5-Dihydro-1H-pyrazol-4-yl, 4,5-Dihydro-1H-pyrazol-5-yl, 2,5-Dihydro-1H-pyrazol-3-yl, 2,5-Dihydro-1H-pyrazol-4-yl, 2,5-Dihydro-1H-pyrazol-5-yl, 4,5-Dihydroisoxazol-3-yl, 4,5-Dihydroisoxazol-4-yl, 4,5-Dihydroisoxazol-5-yl, 2,5-Dihydroisoxazol-3-yl, 2,5-Dihydroisoxazol-4-yl, 2,5-Dihydroisoxazol-5-yl, 2,3-Dihydroisoxazol-3-yl, 2,3-Dihydroisoxazol-4-yl, 2,3-Dihydroisoxazol-5-yl, 4,5-Dihydroisothiazol-3-yl, 4,5-Dihydroisothiazol-4-yl, 4,5-Dihydroisothiazol-5-yl, 2,5-Dihydroisothiazol-3-yl, 2,5-Dihydroisothiazol-4-yl, 2,5-Dihydroisothiazol-5-yl, 2,3-Dihydroisothiazol-3-yl, 2,3-Dihydroisothiazol-4-yl, 2,3-Dihydroisothiazol-5-yl, Δ³-1,2-Dithiol-3-yl, Δ³-1,2-Dithiol-4-yl, Δ³-1,2-Dithiol-5-yl, 4,5-Dihydro-1H-imidazol-2-yl, 4,5-Dihydro-1H-imidazol-4-yl, 4,5-Dihydro-1H-imidazol-5-yl, 2,5-Dihydro-1H-imidazol-2-yl, 2,5-Dihydro-1H-imidazol-4-yl, 2,5-Dihydro-1H-imidazol-5-yl, 2,3-Dihydro-1H-imidazol-2-yl, 2,3-Dihydro-1H-imidazol-4-yl, 4,5-Dihydrooxazol-2-yl, 4,5-Dihydrooxazol-4-yl, 4,5-Dihydrooxazol-5-yl, 2,5-Dihydrooxazol-2-yl, 2,5-Dihydrooxazol-4-yl, 2,5-Dihydrooxazol-5-yl, 2,3-Dihydrooxazol-2-yl, 2,3-Dihydrooxazol-4-yl, 2,3-Dihydrooxazol-5-yl, 4,5-Dihydrothiazol-2-yl, 4,5-Dihydrothiazol-4-yl, 4,5-Dihydrothiazol-5-yl, 2,5-Dihydrothiazol-2-yl, 2,5-Dihydrothiazol-4-yl, 2,5-Dihydrothiazol-5-yl, 2,3-Dihydrothiazol-2-yl, 2,3-Dihydrothiazol-4-yl, 2,3-Dihydrothiazol-5-yl, 1,3-Dioxol-2-yl, 1,3-Dioxol-4-yl, 1,3-Dithiol-2-yl, 1,3-Dithiol-4-yl, 1,3-Oxathiol-2-yl, 1,3-Oxathiol-4-yl, 1,3-Oxathiol-5-yl, 1,2,3-Δ²-Oxadiazolin-4-yl, 1,2,3-Δ²-Oxadiazolin-5-yl, 1,2,4-Δ⁴-Oxadiazolin-3-yl, 1,2,4-Δ⁴-oxadiazolin-5-yl, 1,2,4-Δ²-Oxadiazolin-3-yl, 1,2,4-Δ²-Oxadiazolin-5-yl, 1,2,4-Δ³-Oxadiazolin-3-yl, 1,2,4-Δ³-Oxadiazolin-5-yl, 1,3,4-Δ²-Oxadiazolin-2-yl, 1,3,4-Δ²-Oxadiazolin-5-yl, 1,3,4-Δ³-Oxadiazolin-2-yl, 1,3,4-oxadiazolin-2-yl, 1,2,4-Δ⁴-Thiadiazolin-3-yl, 1,2,4-Δ⁴-Thiadiazolin-5-yl, 1,2,4-Δ³-Thiadiazolin-3-yl, 1,2,4-Δ³-Thiadiazolin-5-yl, 1,2,4-Δ²-Thiadiazolin-3-yl, 1,2,4-Δ²-Thiadiazolin-5-yl, 1,3,4-Δ²-Thiadiazolin-2-yl, 1,3,4-Δ²-Thiadiazolin-5-yl, 1,3,4-Δ³-Thiadiazolin-2-yl, 1,3,4-Thiadiazolin-2-yl, 1,3,2-Dioxathiolan-4-yl, 1,2,3-Δ²-Triazolin-4-yl, 1,2,3-Δ²-Triazolin-5-yl, 1,2,4-Δ²-Triazolin-3-yl, 1,2,4-Δ²-Triazolin-5-yl, 1,2,4-Δ³-Triazolin-3-yl, 1,2,4-Δ³-Triazolin-5-yl, 1,2,4-Δ¹-Triazolin-2-yl, 1,2,4-Triazolin-3-yl, Tetrahydropyran-2-yl, Tetrahydropyran-3-yl, Tetrahydropyran-4-yl, Piperidin-2-yl, Piperidin-3-yl, Piperidin-4-yl, Tetrahydrothiopyran-2-yl, Tetrahydrothiopyran-3-yl, Tetrahydrothiopyran-4-yl, 2H-3,4-Dihydropyran-6-yl, 2H-3,4-Dihydropyran-5-yl, 2H-3,4-Dihydropyran-4-yl, 2H-3,4-Dihydropyran-3-yl, 2H-3,4-Dihydropyran-2-yl, 2H-3,4-Dihydrothiopyran-6-yl, 2H-3,4-Dihydrothiopyran-5-yl, 2H-3,4-Dihydrothiopyran-4-yl, 2H-3,4-Dihydrothiopyran-3-yl, 2H-3,4-Dihydrothiopyran-2-yl, 1,2,3,4-Tetrahydropyridin-6-yl, 1,2,3,4-Tetrahydropyridin-5-yl, 1,2,3,4-Tetrahydropyridin-4-yl, 1,2,3,4-Tetrahydropyridin-3-yl, 1,2,3,4-Tetrahydropyridin-2-yl, 2H-5,6-Dihydropyran-2-yl, 2H-5,6-Dihydropyran-3-yl, 2H-5,6-Dihydropyran-4-yl, 2H-5,6-Dihydropyran-5-yl, 2H-5,6-Dihydropyran-6-yl, 2H-5,6-Dihydrothiopyran-2-yl, 2H-5,6-Dihydrothiopyran-3-yl, 2H-5,6-Dihydrothiopyran-4-yl, 2H-5,6-Dihydrothiopyran-5-yl, 2H-5,6-Dihydrothiopyran-6-yl, 1,2,5,6-Tetrahydropyridin-2-yl, 1,2,5,6-Tetrahydropyridin-3-yl, 1,2,5,6-Tetrahydropyridin-4-yl, 1,2,5,6-Tetrahydropyridin-5-yl, 1,2,5,6-Tetrahydropyridin-6-yl, 2,3,4,5-Tetrahydropyridin-2-yl, 2,3,4,5-Tetrahydropyridin-3-yl, 2,3,4,5-Tetrahydropyridin-4-yl, 2,3,4,5-Tetrahydropyridin-5-yl, 2,3,4,5-Tetrahydropyridin-6-yl, 4H-Pyran-2-yl, 4H-Pyran-3-yl, 4H-Pyran-4-yl, 4H-Thiopyran-2-yl, 4H-Thiopyran-3-yl, 4H-Thiopyran-4-yl, 1,4-Dihydropyridin-2-yl, 1,4-Dihydropyridin-3-yl, 1,4-Dihydropyridin-4-yl, 2H-Pyran-2-yl, 2H-Pyran-3-yl, 2H-Pyran-4-yl, 2H-Pyran-5-yl, 2H-Pyran-6-yl, 2H-Thiopyran-2-yl, 2H-Thiopyran-3-yl, 2H-Thiopyran-4-yl, 2H-Thiopyran-5-yl, 2H-Thiopyran-6-yl, 1,2-Dihydropyridin-2-yl, 1,2-Dihydropyridin-3-yl, 1,2-Dihydropyridin-4-yl, 1,2-Dihydropyridin-5-yl, 1,2-Dihydropyridin-6-yl, 3,4-Dihydropyridin-2-yl, 3,4-Dihydropyridin-3-yl, 3,4-Dihydropyridin-4-yl, 3,4-Dihydropyridin-5-yl, 3,4-Dihydropyridin-6-yl, 2,5-Dihydropyridin-2-yl, 2,5-Dihydropyridin-3-yl, 2,5-Dihydropyridin-4-yl, 2,5-Dihydropyridin-5-yl, 2,5-Dihydropyridin-6-yl, 2,3-Dihydropyridin-2-yl, 2,3-Dihydropyridin-3-yl, 2,3-Dihydropyridin-4-yl, 2,3-Dihydropyridin-5-yl, 2,3-Dihydropyridin-6-yl, 1,3-Dioxan-4-yl, 1,3-Dioxan-5-yl, 1,4-Dioxan-2-yl, 1,3-Dithian-2-yl, 1,3-Dithian-4-yl, 1,3-Dithian-5-yl, 1,4-Dithian-2-yl, 1,3-Oxathian-2-yl, 1,3-Oxathian-4-yl, 1,3-Oxathian-5-yl, 1,3-Oxathian-6-yl, 1,4-Oxathian-2-yl, 1,4-Oxathian-3-yl, 1,2-Dithian-3-yl, 1,2-Dithian-4-yl, Hexahydropyrimidin-2-yl, Hexahydropyrimidin-4-yl, Hexahydropyrimidin-5-yl, Hexahydropyrazin-2-yl, Hexahydropyridazin-3-yl, Hexahydropyridazin-4-yl, Tetrahydro-1,3-oxazin-2-yl, Tetrahydro-1,3-oxazin-4-yl, Tetrahydro-1,3-oxazin-5-yl, Tetrahydro-1,3-oxazin-6-yl, Tetrahydro-1,3-thiazin-2-yl, Tetrahydro-1,3-thiazin-4-yl, Tetrahydro-1,3-thiazin-5-yl, Tetrahydro-1,3-thiazin-6-yl, Tetrahydro-1,4-thiazin-2-yl, Tetrahydro-1,4-thiazin-3-yl, Tetrahydro-1,4-oxazin-2-yl, Tetrahydro-1,4-oxazin-3-yl, Tetrahydro-1,2-oxazin-3-yl, Tetrahydro-1,2-oxazin-4-yl, Tetrahydro-1,2-oxazin-5-yl, Tetrahydro-1,2-oxazin-6-yl, 2H-5,6-Dihydro-1,2-oxazin-3-yl, 2H-5,6-Dihydro-1,2-oxazin-4-yl, 2H-5,6-Dihydro-1,2-oxazin-5-yl, 2H-5,6-Dihydro-1,2-oxazin-6-yl, 2H-5,6-Dihydro-1,2-thiazin-3-yl, 2H-5,6-Dihydro-1,2-thiazin-4-yl, 2H-5,6-Dihydro-1,2-thiazin-5-yl, 2H-5,6-Dihydro-1,2-thiazin-6-yl, 4H-5,6-Dihydro-1,2-oxazin-3-yl, 4H-5,6-Dihydro-1,2-oxazin-4-yl, 4H-5,6-Dihydro-1,2-oxazin-5-yl, 4H-5,6-Dihydro-1,2-oxazin-6-yl, 4H-5,6-Dihydro-1,2-thiazin-3-yl, 4H-5,6-Dihydro-1,2-thiazin-4-yl, 4H-5,6-Dihydro-1,2-thiazin-5-yl, 4H-5,6-Dihydro-1,2-thiazin-6-yl, 2H-3,6-Dihydro-1,2-oxazin-3-yl, 2H-3,6-Dihydro-1,2-oxazin-4-yl, 2H-3,6-Dihydro-1,2-oxazin-5-yl, 2H-3,6-Dihydro-1,2-oxazin-6-yl, 2H-3,6-Dihydro-1,2-thiazin-3-yl, 2H-3,6-Dihydro-1,2-thiazin-4-yl, 2H-3,6-Dihydro-1,2-thiazin-5-yl, 2H-3,6-Dihydro-1,2-thiazin-6-yl, 2H-3,4-Dihydro-1,2-oxazin-3-yl, 2H-3,4-Dihydro-1,2-oxazin-4-yl, 2H-3,4-Dihydro-1,2-oxazin-5-yl, 2H-3,4-Dihydro-1,2-oxazin-6-yl, 2H-3,4-Dihydro-1,2-thiazin-3-yl, 2H-3,4-Dihydro-1,2-thiazin-4-yl, 2H-3,4-Dihydro-1,2-thiazin-5-yl, 2H-3,4-Dihydro-1,2-thiazin-6-yl, 2,3,4,5-Tetrahydropyridazin-3-yl, 2,3,4,5-Tetrahydropyridazin-4-yl, 2,3,4,5-Tetrahydropyridazin-5-yl, 2,3,4,5-Tetrahydropyridazin-6-yl, 3,4,5,6-Tetrahydropyridazin-3-yl, 3,4,5,6-Tetrahydropyridazin-4-yl, 1,2,5,6-Tetrahydropyridazin-3-yl, 1,2,5,6-Tetrahydropyridazin-4-yl, 1,2,5,6-Tetrahydropyridazin-5-yl, 1,2,5,6-Tetrahydropyridazin-6-yl, 1,2,3,6-Tetrahydropyridazin-3-yl, 1,2,3,6-Tetrahydropyridazin-4-yl, 4H-5,6-Dihydro-1,3-oxazin-2-yl, 4H-5,6-Dihydro-1,3-oxazin-4-yl, 4H-5,6-Dihydro-1,3-oxazin-5-yl, 4H-5,6-Dihydro-1,3-oxazin-6-yl, 4H-5,6-Dihydro-1,3-thiazin-2-yl, 4H-5,6-Dihydro-1,3-thiazin-4-yl, 4H-5,6-Dihydro-1,3-thiazin-5-yl, 4H-5,6-Dihydro-1,3-thiazin-6-yl, 3,4,5,6-Tetrahydropyrimidin-2-yl, 3,4,5,6-Tetrahydropyrimidin-4-yl, 3,4,5,6-Tetrahydropyrimidin-5-yl, 3,4,5,6-Tetrahydropyrimidin-6-yl, 1,2,3,4-Tetrahydropyrazin-2-yl, 1,2,3,4-Tetrahydropyrazin-5-yl, 1,2,3,4-Tetrahydropyrimidin-2-yl, 1,2,3,4-Tetrahydropyrimidin-4-yl, 1,2,3,4-Tetrahydropyrimidin-5-yl, 1,2,3,4-Tetrahydropyrimidin-6-yl, 2,3-Dihydro-1,4-thiazin-2-yl, 2,3-Dihydro-1,4-thiazin-3-yl, 2,3-Dihydro-1,4-thiazin-5-yl, 2,3-Dihydro-1,4-thiazin-6-yl, 2H-1,2-Oxazin-3-yl, 2H-1,2-Oxazin-4-yl, 2H-1,2-Oxazin-5-yl, 2H-1,2-Oxazin-6-yl, 2H-1,2-Thiazin-3-yl, 2H-1,2-Thiazin-4-yl, 2H-1,2-Thiazin-5-yl, 2H-1,2-Thiazin-6-yl, 4H-1,2-Oxazin-3-yl, 4H-1,2-Oxazin-4-yl, 4H-1,2-Oxazin-5-yl, 4H-1,2-Oxazin-6-yl, 4H-1,2-Thiazin-3-yl, 4H-1,2-Thiazin-4-yl, 4H-1,2-Thiazin-5-yl, 4H-1,2-Thiazin-6-yl, 6H-1,2-Oxazin-3-yl, 6H-1,2-Oxazin-4-yl, 6H-1,2-Oxazin-5-yl, 6H-1,2-Oxazin-6-yl, 6H-1,2-Thiazin-3-yl, 6H-1,2-Thiazin-4-yl, 6H-1,2-Thiazin-5-yl, 6H-1,2-Thiazin-6-yl, 2H-1,3-Oxazin-2-yl, 2H-1,3-Oxazin-4-yl, 2H-1,3-Oxazin-5-yl, 2H-1,3-Oxazin-6-yl, 2H-1,3-Thiazin-2-yl, 2H-1,3-Thiazin-4-yl, 2H-1,3-Thiazin-5-yl, 2H-1,3-Thiazin-6-yl, 4H-1,3-Oxazin-2-yl, 4H-1,3-Oxazin-4-yl, 4H-1,3-Oxazin-5-yl, 4H-1,3-Oxazin-6-yl, 4H-1,3-Thiazin-2-yl, 4H-1,3-Thiazin-4-yl, 4H-1,3-Thiazin-5-yl, 4H-1,3-Thiazin-6-yl, 6H-1,3-Oxazin-2-yl, 6H-1,3-Oxazin-4-yl, 6H-1,3-Oxazin-5-yl, 6H-1,3-Oxazin-6-yl, 6H-1,3-Thiazin-2-yl, 6H-1,3-Oxazin-4-yl, 6H-1,3-Oxazin-5-yl, 6H-1,3-Thiazin-6-yl, 2H-1,4-Oxazin-2-yl, 2H-1,4-Oxazin-3-yl, 2H-1,4-Oxazin-5-yl, 2H-1,4-Oxazin-6-yl, 2H-1,4-Thiazin-2-yl, 2H-1,4-Thiazin-3-yl, 2H-1,4-Thiazin-5-yl, 2H-1,4-Thiazin-6-yl, 4H-1,4-Oxazin-2-yl, 4H-1,4-Oxazin-3-yl, 4H-1,4-Thiazin-2-yl, 4H-1,4-Thiazin-3-yl, 1,4-Dihydropyridazin-3-yl, 1,4-Dihydropyridazin-4-yl, 1,4-Dihydropyridazin-5-yl, 1,4-Dihydropyridazin-6-yl, 1,4-Dihydropyrazin-2-yl, 1,2-Dihydropyrazin-2-yl, 1,2-Dihydropyrazin-3-yl, 1,2-Dihydropyrazin-5-yl, 1,2-Dihydropyrazin-6-yl, 1,4-Dihydropyrimidin-2-yl, 1,4-Dihydropyrimidin-4-yl, 1,4-Dihydropyrimidin-5-yl, 1,4-Dihydropyrimidin-6-yl, 3,4-Dihydropyrimidin-2-yl, 3,4-Dihydropyrimidin-4-yl, 3,4-Dihydropyrimidin-5-yl, 3,4-Dihydropyrimidin-6-yl,
wobei der Schwefel der genannten Heterocyclen zu S=O oder S(=O)₂ oxidiert sein kann
und wobei der Fünfring oder Sechsring unsubstituiert ist.

Ebenso in Hinblick auf die biologische Wirkung bevorzugt sind Carbamoyltriazole der Formel I wobei R¹ bis R⁵ und A die für die voranstehend aufgeführten Verbindungen genannten Bedeutungen haben und A zusätzlich 1,3-Dioxolan-2-yl und 1,3-Dioxan-2-yl sein kann und wobei der Schwefel der genannten Heterocyclen zu S=O oder S(=O)₂ oxidiert sein kann
und
wobei ein oben genannter Fünfringheterocyclus ein bis drei an Kohlenstoff gebundene Reste, ausgewählt aus der Gruppe R⁶, R⁷ und R⁸ trägt und wobei jeder Ringstickstoff unabhängig voneinander einen Rest R¹⁰ tragen kann
und
wobei ein oben genannter Sechsringheterocyclus ein bis vier an Kohlenstoff gebundene Reste, ausgewählt aus der Gruppe R⁶, R⁷, R⁸ und R⁹ trägt und wobei jeder Ringstickstoff unabhängig voneinander einen Rest R¹⁰ tragen kann;
- R⁶,R⁷,R⁸,R⁹: Cyano, Halogen, C₁-C₆-Alkyl, C₁-C₆-Halogenalkyl, C₃-C₆-Cycloalkyl, C₂-C₆-Alkenyl, C₂-C₆-Alkinyl, C₁-C₆-Alkoxy, C₁-C₆-Halogenalkoxy, C₁-C₆-Alkylthio, C₁-C₆-Halogenalkylthio, C₁-C₆-Alkylcarbonyl, C₁-C₆-Halogenalkylcarbonyl, C₁-C₆-Alkoxycarbonyl, Phenyl oder Benzyl, wobei die beiden letztgenannten Substituenten partiell oder vollständig halogeniert sein können und eine bis drei der folgenden Gruppen tragen können:
Nitro, Cyano, C₁-C₄-Alkyl, C₁-C₄-Halogenalkyl, C₁-C₄-Alkoxy, C₁-C₄-Halogenalkoxy und C₁-C₄-Alkoxycarbonyl;
- R¹⁰: C₁-C₆-Alkyl, C₁-C₆-Halogenalkyl, C₃-C₆-Cycloalkyl, C₃-C₆-Alkenyl, C₃-C₆-Alkinyl, C₁-C₆-Alkylcarbonyl, C₁-C₆-Halogenalkylcarbonyl, C₁-C₆-Alkoxycarbonyl, Phenyl oder Benzyl, wobei die beiden letztgenannten Substituenten partiell oder vollständig halogeniert sein können und/oder eine bis drei der folgenden Gruppen tragen können:
Nitro, Cyano, C₁-C₄-Alkyl, C₁-C₄-Halogenalkyl, C₁-C₄-Alkoxy, C₁-C₄-Halogenalkoxy und C₁-C₄-Alkoxycarbonyl.

Außerordentlich bevorzugt sind die substituierten Carbamoyltriazole der Formel Ia (≙ I mit n=0), insbesondere die Verbindungen der Tabelle 1:

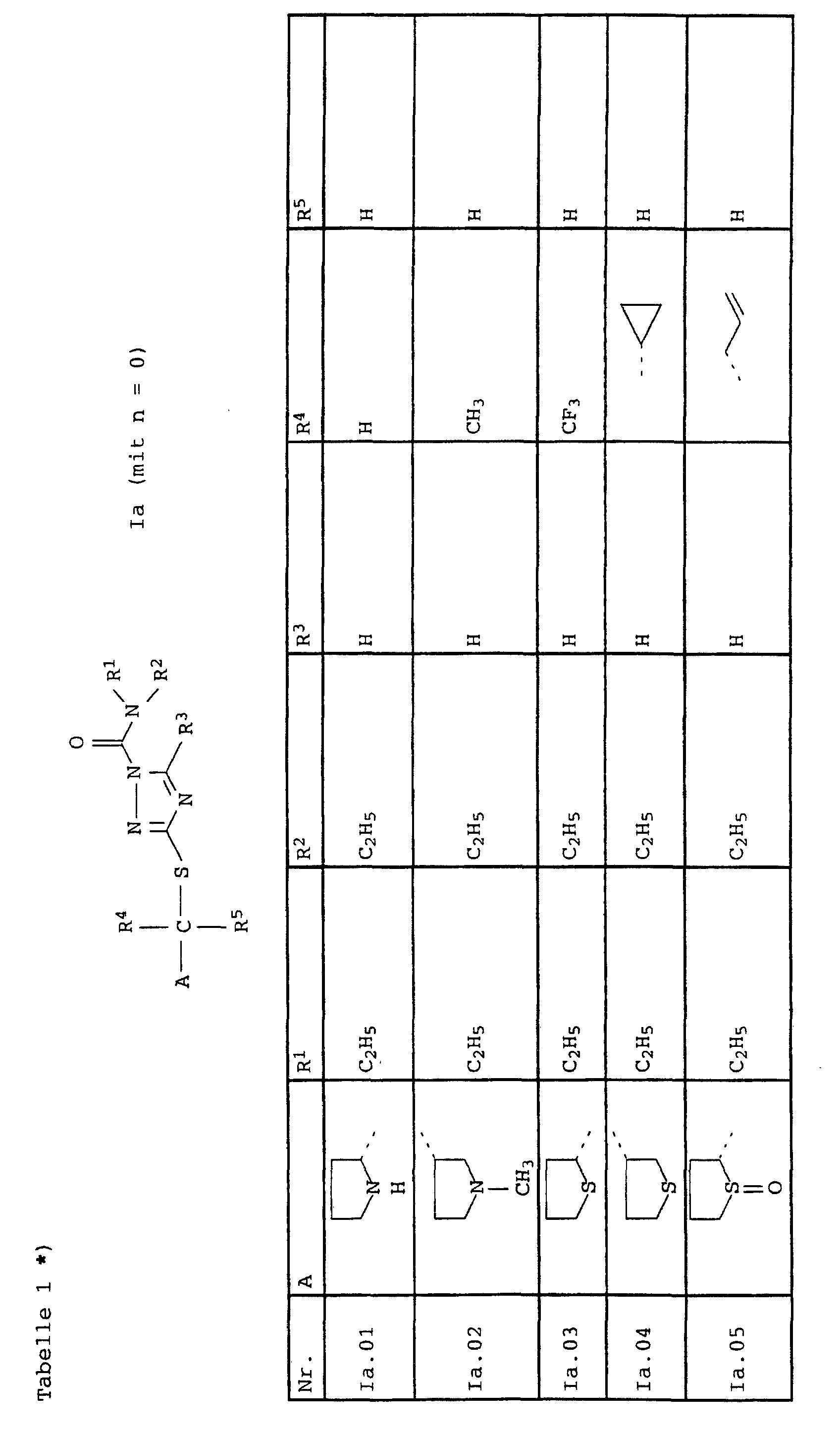

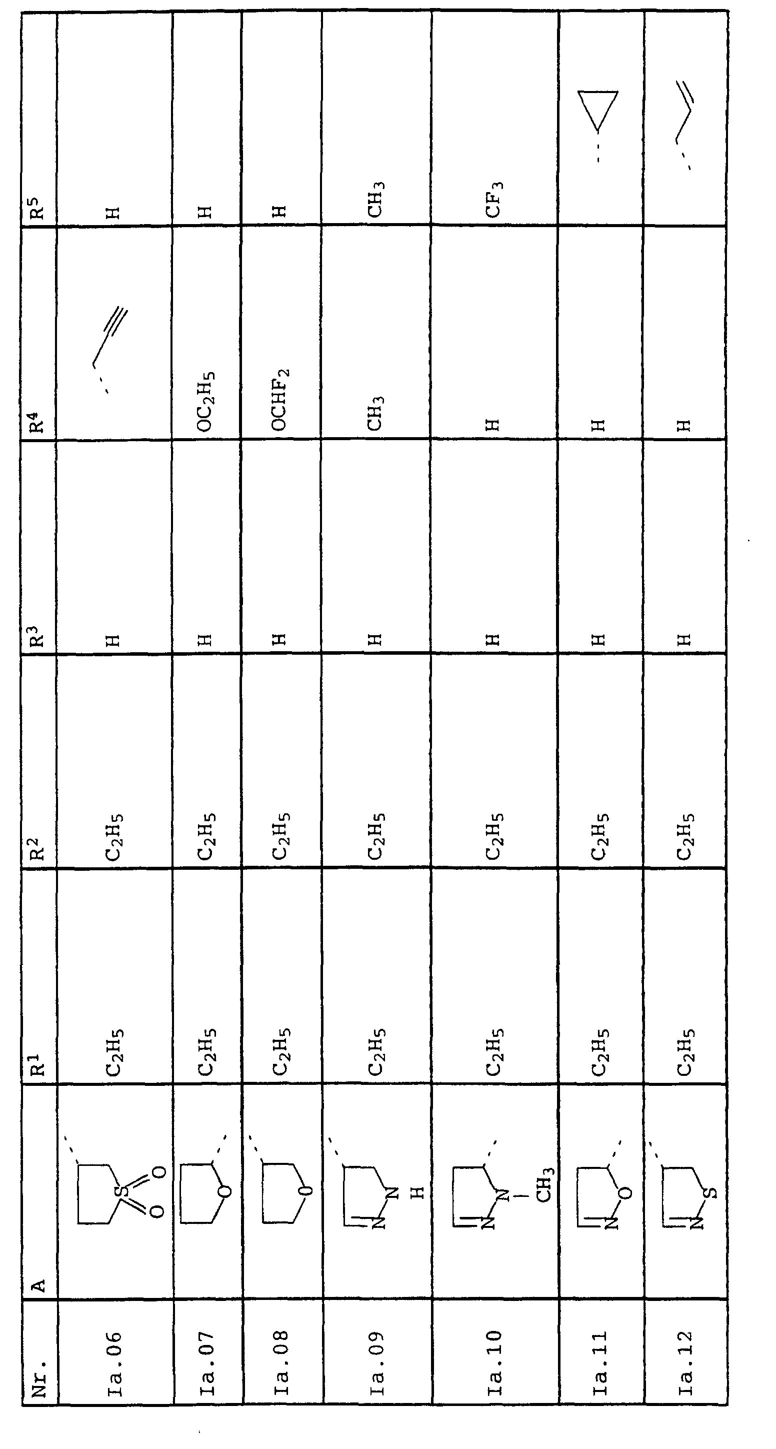

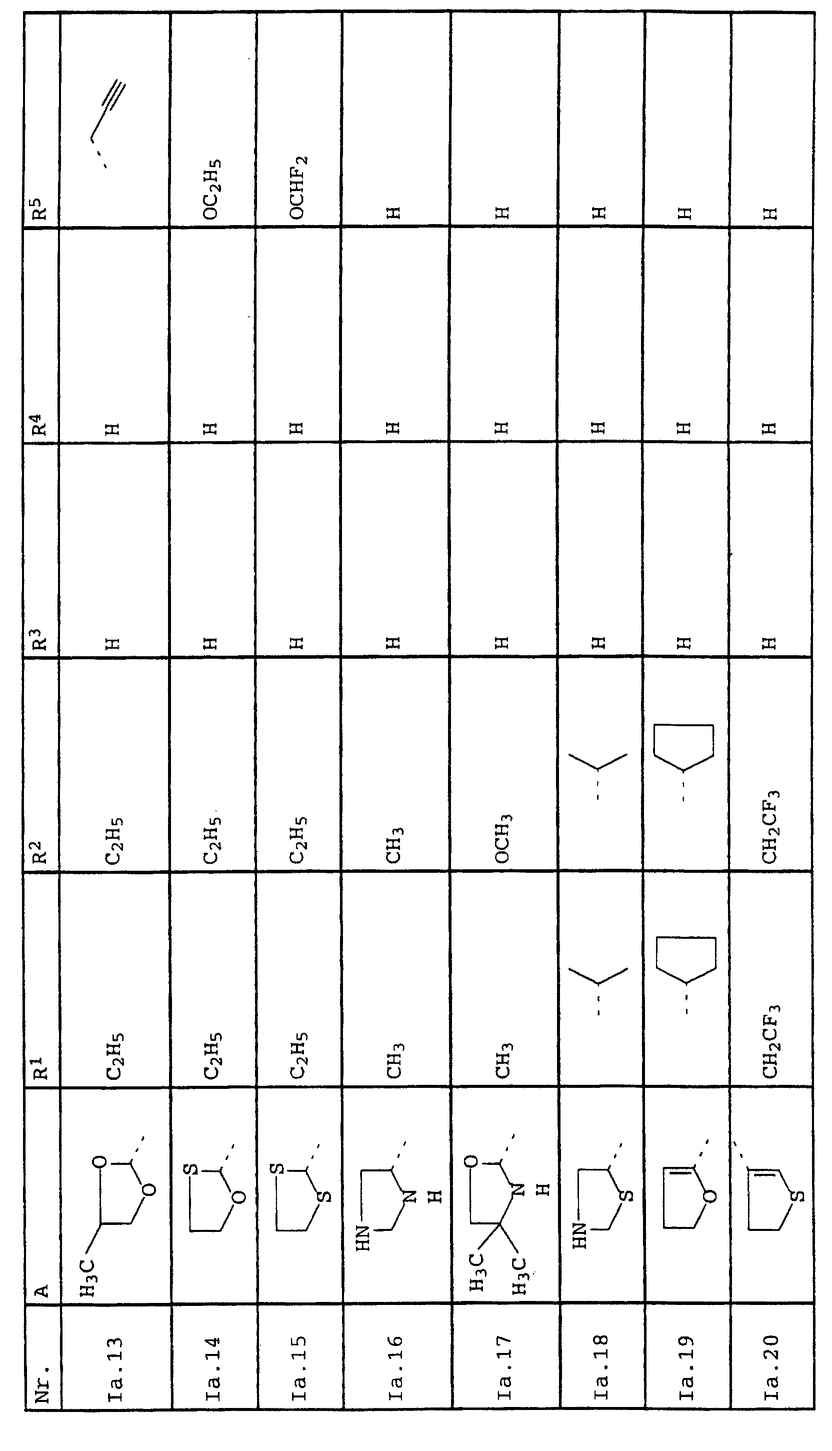

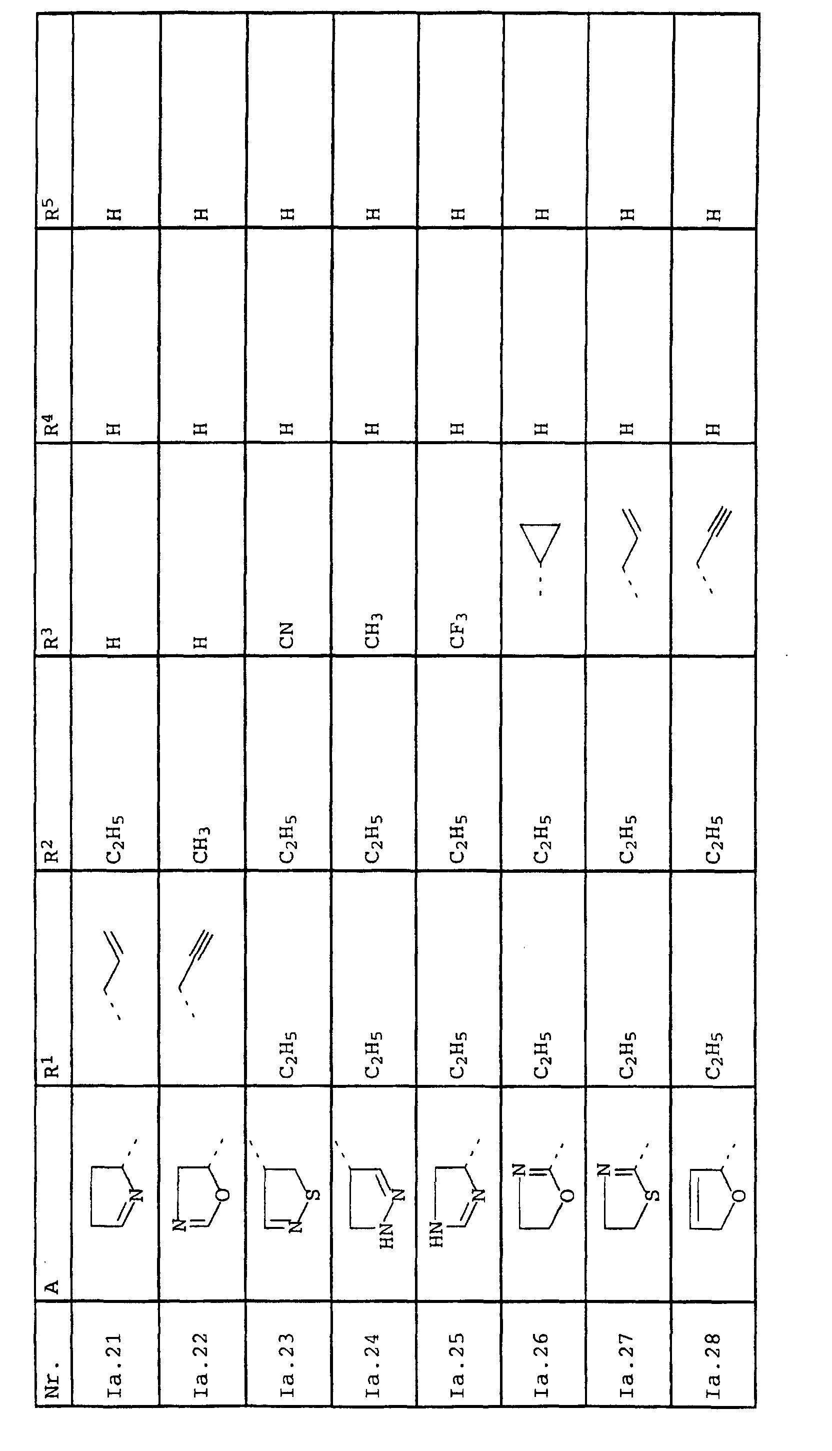

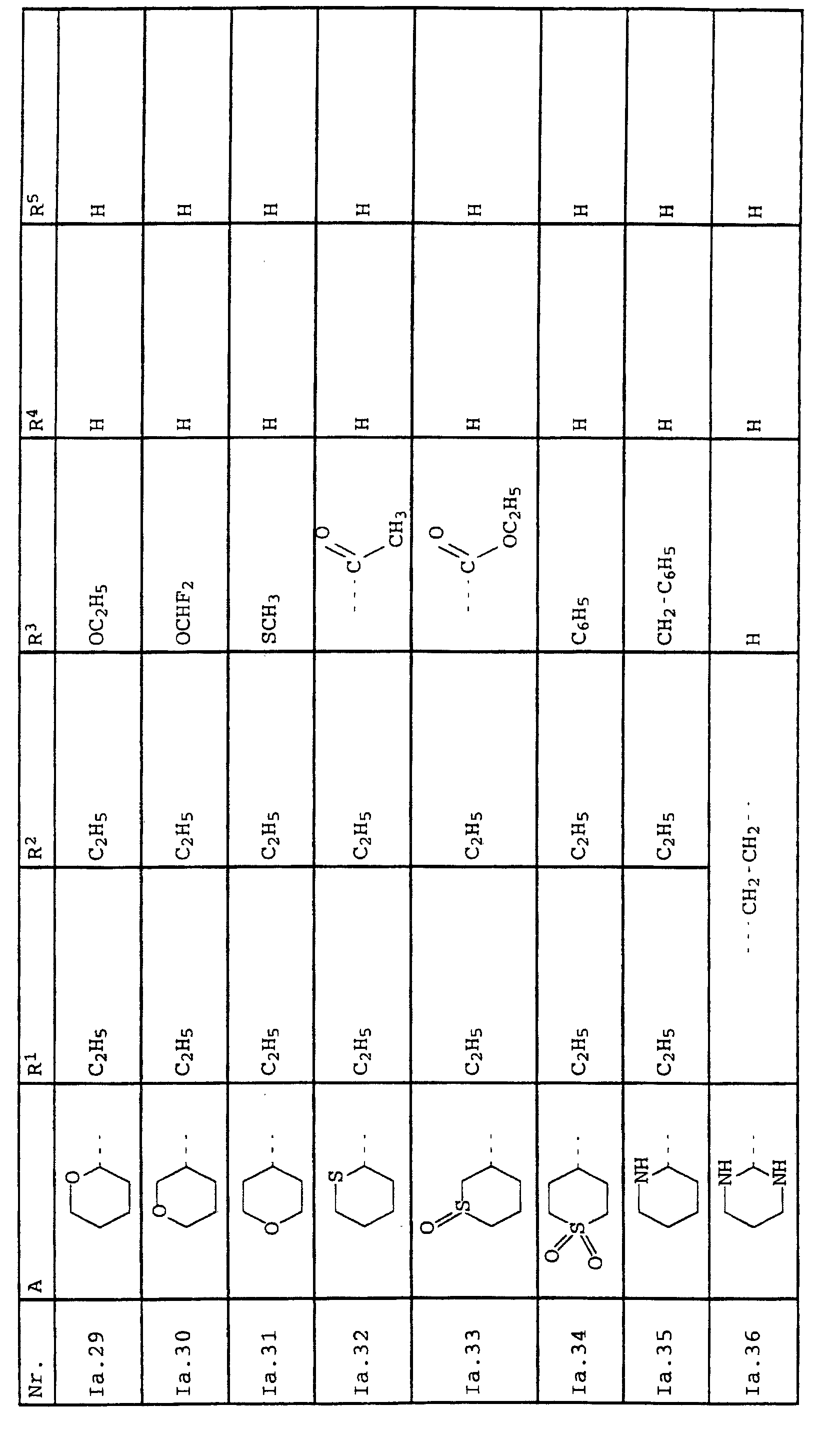

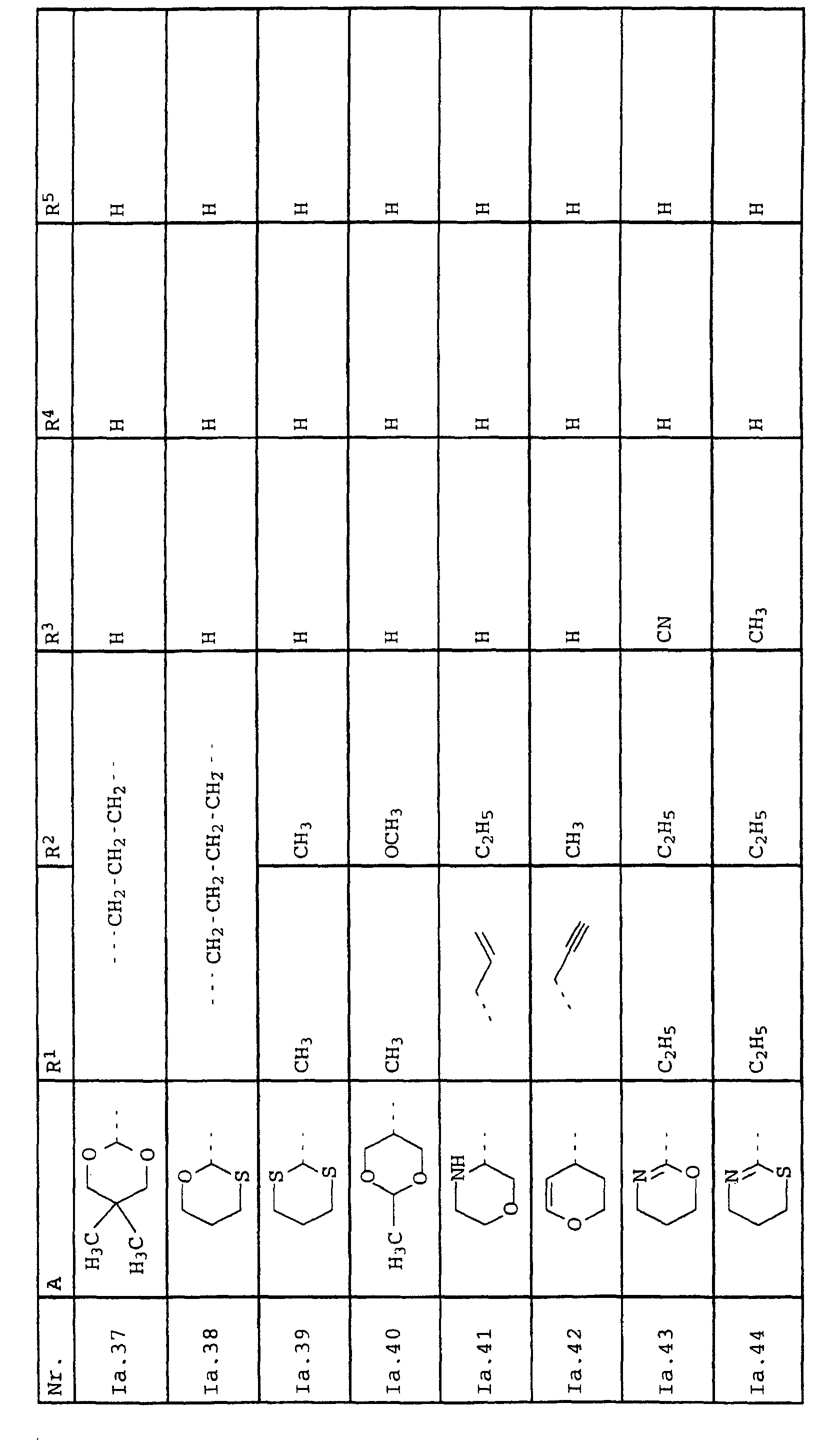

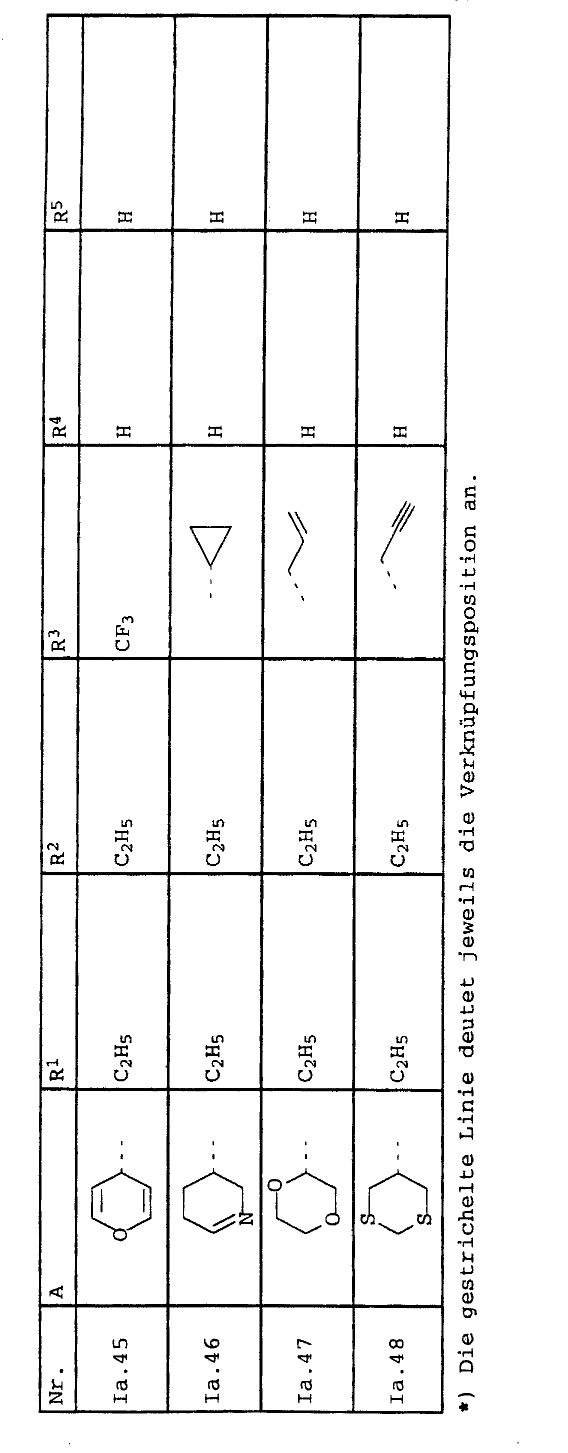

- Des weiteren sind die substituierten Carbamoyltriazole der Formel Ib, insbesondere die Verbindungen Ib.01-Ib.48, die sich von den Verbindungen Ia.01-Ia.48 dadurch unterscheiden, daß n für 1 steht, außerordentlich bevorzugt: 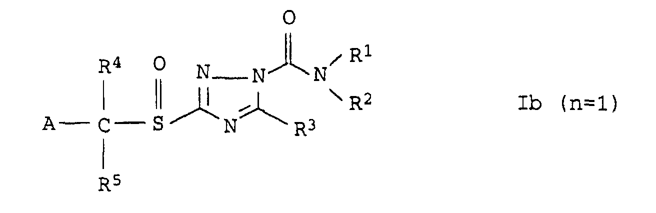
- Ebenso sind die substituierten Carbamoyltriazole der Formel Ic, insbesondere die Verbindungen Ic.01-Ic.48, die sich von den Verbindungen Ia.01-Ia.48 dadurch unterscheiden, daß n für 2 steht, außerordentlich bevorzugt:

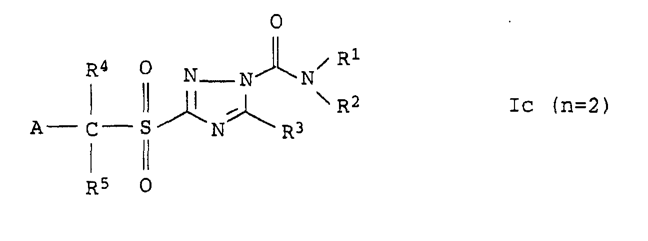

Ganz außerordentlich bevorzugt sind die substituierten Carbamoyltriazole der Formel Ic, insbesondere die Verbindungen Ic.01-Ic.48 wie voranstehend aufgeführt.

Außerordentlich bevorzugt sind ferner substituierte Carbamoyltriazole der Formel I, wobei R¹ und R² jeweils C₁-C₆-Alkyl bedeuten.

Ebenso außerordentlich bevorzugt sind substituierte Carbamoyltriazole der Formel I, wobei R³ und R⁴ jeweils Wasserstoff bedeuten.

Außerordentlich bevorzugt sind ferner substituierte Carbamoyltriazole der Formel I, wobei A für einen ggf. substituierten, gesättigten oder partiell ungesättigten Heterocyclus, der ein Heteroatom enthält und fünf- oder sechsgliedrig ist, steht, also z.B. Tetrahydrofuran-2-yl, Tetrahydrofuran-3-yl, Tetrahydrothien-2-yl, Tetrahydrothien-3-yl, Tetrahydropyrrol-2-yl, Tetrahydropyrrol-3-yl, 2,3-Dihydrofuran-2-yl, 2,3-Dihydrofuran-3-yl, 2,5-Dihydrofuran-2-yl, 2,5-Dihydrofuran-3-yl, 4,5-Dihydrofuran-2-yl, 4,5-Dihydrofuran-3-yl, 2,3-Dihydrothienyl-2-yl, 2,3-Dihydrothien-3-yl, 2,5-Dihydrothien-2-yl, 2,5-Dihydrothien-3-yl, 4,5-Dihydrothien-2-yl, 4,5-Dihydrothien-3-yl, 2,3-Dihydro-1H-pyrrol-2-yl, 2,3-Dihydro-1H-pyrrol-3-yl, 2,5.Dihydro-1H-pyrrol-2-yl, 2,5-Dihydro-1H-pyrrol-3-yl, 4,5-Dihydro-1H-pyrrol-2-yl, 4,5-Dihydro-1H-pyrrol-3-yl, 3,4-Dihydro-2H-pyrrol-2-yl, 3,4-Dihydro-2H-pyrrol-3-yl, 3,4-Dihydro-2H-pyrrol-4-yl, 3,4-Dihydro-2H-pyrrol-5-yl, Tetrahydropyran-2-yl, Tetrahydropyran-3-yl, Tetrahydropyran-4-yl, Tetrahydrothiopyran-2-yl, Tetrahydrothiopyran-3-yl, Tetrahydrothiopyran-4-yl, Piperdin-2-yl, Piperidin-3-yl, Piperidin-4-yl, 2H-3,4-Dihydropyran-6-yl, 2H-3,4-Dihydropyran-5-yl, 2H-3,4-Dihydropyran-4-yl, 2H-3,4-Dihydropyran-3-yl, 2H-3,4-Dihydropyran-2-yl, 2H-5,6-Dihydropyran-2-yl, 2H-5,6-Dihydropyran-3-yl, 2H-5,6-Dihydropyran-4-yl, 2H-5,6-Dihydropyran-5-yl, 2H-5,6-Dihydropyran-6-yl, 2H-3,4-Dihydrothiopyran-6-yl, 2H-3,4-Dihydrothiopyran-5-yl, 2H-3,4-Dihydrothiopyran-4-yl, 2H-3,4-Dihydrothiopyran-3-yl, 2H-3,4-Dihydrothiopyran-2-yl, 2H-5,6-Dihydrothiopyran-2-yl, 2H-5,6-Dihydrothiopyran-3-yl, 2H-5,6-Dihydrothiopyran-4-yl, 2H-5,6-Dihydrothiopyran-5-yl, 2H-5,6-Dihydrothiopyran-6-yl, 1,2,3,4-Tetrahydropyridin-6-yl, 1,2,3,4-Tetrahydropyridin-5-yl, 1,2,3,4-Tetrahydropyridin-4-yl, 1,2,3,4-Tetrahydropyridin-3-yl, 1,2,3,4-Tetrahydropyridin-2-yl, 1,2,5,6-Tetrahydropyridin-2-yl, 1,2,5,6-Tetrahydropyridin-3-yl, 1,2,5,6-Tetrahydropyridin-4-yl, 1,2,5,6-Tetrahydropyridin-5-yl, 1,2,5,6-Tetrahydropyridin-6-yl, 2,3,4,5-Tetrahydropyridin-2-yl, 2,3,4,5-Tetrahydropyridin-3-yl, 2,3,4,5-Tetrahydropyridin-4-yl, 2,3,4,5-Tetrahydropyridin-5-yl, 2,3,4,5-Tetrahydropyridin-6-yl, 4H-Pyran-2-yl, 4H-Pyran-3-yl, 4H-Pyran-4-yl, 2H-Pyran-2-yl, 2H-Pyran-3-yl, 2H-Pyran-4-yl, 2H-Pyran-5-yl, 2H-Pyran-6-yl, 4H-Thiopyran-2-yl, 4H-Thiopyran-3-yl, 4H-Thiopyran-4-yl, 2H-Thiopyran-2-yl, 2H-Thiopyran-3-yl, 2H-Thiopyran-4-yl, 2H-Thiopyran-5-yl, 2H-Thiopyran-6-yl, 1,4-Dihydropyridin-2-yl, 1,4-Dihydropyridin-3-yl, 1,4-Dihydropyridin-4-yl, 1,2-Dihydropyridin-2-yl, 1,2-Dihydropyridin-3-yl, 1,2-Dihydropyridin-3-yl, 1,2-Dihydropyridin-4-yl, 1,2-Dihydropyridin-5-yl, 1,2-Dihydropyridin-6-yl, 3,4-Dihydropyridin-2-yl, 3,4-Dihydropyridin-3-yl, 3,4-Dihydropyridin-4-yl, 3,4-Dihydropyridin-5-yl, 3,4-Dihydropyridin-6-yl, 2,5-Dihydropyridin-2-yl, 2,5-Dihydropyridin-3-yl, 2,5-Dihydropyridin-4-yl, 2,5-Dihydropyridin-5-yl, 2,5-Dihydropyridin-6-yl, 2,3-Dihydropyridin-2-yl, 2,3-Dihydropyridin-3-yl, 2,3-Dihydropyridin-4-yl, 2,3-Dihydropyridin-5-yl, 2,3-Dihydropyridin-6-yl, wobei die voranstehend genannten Fünfringheterocyclen einen bis drei an Kohlenstoff gebundene Reste, ausgewählt aus der Gruppe R⁶, R⁷ und R⁸, und ggf. der Ringstickstoff den Rest R¹⁰ tragen können und wobei die voranstehenden Sechsringheterocyclen einen bis vier an Kohlenstoff gebundene Reste, ausgewählt aus der Gruppe R⁶, R⁷, R⁸ und R⁹, und ggf. der Ringstickstoff den Rest R¹⁰ tragen können und/oder wobei ggf. der Schwefel der genannten Heterocyclen zu S=O oder S(=O)₂ oxidiert sein kann.

Ganz außerordentlich bevorzugt sind substituierte Carbamoyltriazole der Formel I, wobei A für einen ggf. substituierten, gesättigten oder partiell ungesättigten Heterocyclus, der ein Heteroatom, und zwar Sauerstoff, enthält und fünf- oder sechsgliedrig ist, also z.B. Tetrahydrofuran-2-yl, Tetrahydrofuran-3-yl, 2,3-Dihydrofuran-2-yl, 2,3-Dihydrofuran-3-yl, 2,5-Dihydrofuran-2-yl, 2,5-Dihydrofuran-3-yl, 4,5-Dihydrofuran-2-yl, 4,5-Dihydrofuran-3-yl, Tetrahydropyran-2-yl, Tetrahydropyran-3-yl, Tetrahydropyran-4-yl, 2H-3,4-Dihydropyran-6-yl, 2H-3,4-Dihydropyran-5-yl, 2H-3,4-Dihydropyran-4-yl, 2H-3,4-Dihydropyran-3-yl, 2H-3,4-Dihydropyran-2-yl, 2H-5,6-Dihydropyran-2-yl, 2H-5,6-Dihydropyran-3-yl, 2H-5,6-Dihydropyran-4-yl, 2H-5,6-Dihydropyran-5-yl, 2H-5,6-Dihydropyran-6-yl, 4H-Pyran-2-yl, 4H-Pyran-3-yl, 4H-Pyran-4-yl, 2H-Pyran-2-yl 2H-Pyran-3-yl, 2H-Pyran-4-yl, 2H-Pyran-5-yl oder 2H-Pyran-6-yl, wobei die voranstehend genannten Fünfringheterocyclen einen bis drei an Kohlenstoff gebundene Reste, ausgewählt aus der Gruppe R⁶, R⁷ und R⁸, und wobei die voranstehend genannten Sechsringheterocyclen einen bis vier an Kohlenstoff gebundene Reste, ausgewählt aus der Gruppe R⁶, R⁷, R⁸ und R⁹, tragen können.

Außerordentlich bevorzugt sind ferner substituierte Carbamoyltriazole der Formel I, wobei A für einen ggf. substituierten, gesättigten oder partiell ungesättigten Heterocyclus, der zwei Heteroatome enthält und fünf- oder sechsgliedrig ist, steht mit Ausnahme von 1,3-Dioxolan-2-yl und 1,3-Dioxan-2-yl.

Ganz außerordentlich bevorzugt sind substituierte Carbamoyltriazole der Formel I, wobei A für einen ggf. substituierten, gesättigten oder partiell ungesättigten Heterocyclus, der zwei Heteroatome enthält und fünf- oder sechsgliedrig ist, steht, wobei nicht mehr als ein Heteroatom benachbart zur Verknüpfungsposition gebunden ist, also Tetrahydropyrazol-3-yl, Tetrahydropyrazol-4-yl, Tetrahydroisoxazol-3-yl, Tetrahydroisoxazol-4-yl, Tetrahydroisoxazol-5-yl, 1,2-Oxathiolan-3-yl, 1,2-Oxathiolan-4-yl, 1,2-Oxathiolan-5-yl, Tetrahydroisothiazol-3-yl, Tetrahydroisothiazol-4-yl, Tetrahydroisothiazol-5-yl, 1,2-Dithiolan-3-yl, 1,2-Dithiolan-4-yl, Tetrahydroimidazol-4-yl, Tetrahydrooxazol-4-yl, Tetrahydrooxazol-5-yl, Tetrahydrothiazol-4-yl, Tetrahydrothiazol-5-yl, 1,3-Dioxolan-4-yl, 1,3-Oxathiolan-4-yl, 1,3-Oxathiolan-5-yl, 1,3-Dithiolan-4-yl, 4,5-Dihydro-1H-pyrazol-3-yl, 4,5-Dihydro-1H-pyrazol-4-yl, 4,5-Dihydro-1H-pyrazol-5-yl, 2,5-Dihydro-1H-pyrazol-3-yl, 2,5-Dihydro-1H-pyrazol-4-yl, 2,5-Dihydro-1H-pyrazol-5-yl, 4,5-Dihydroisoxazol-3-yl, 4,5-Dihydroisoxazol-4-yl, 4,5-Dihydroisoxazol-5-yl, 2,5-Dihydroisoxazol-3-yl, 2,5-Dihydroisoxazol-4-yl, 2,5-Dihydroisoxazol-5-yl, 2,3-Dihydroisoxazol-3-yl, 2,3-Dihydroisoxazol-4-yl, 2,3-Dihydroisoxazol-5-yl, 4,5-Dihydroisothiazol-3-yl, 4,5-Dihydroisothiazol-4-yl, 4,5-Dihydroisothiazol-5-yl, 2,5-Dihydroisothiazol-3-yl, 2,5-Dihydroisothiazol-4-yl, 2,5-Dihydroisothiazol-5-yl, 2,3-Dihydroisothiazol-3-yl, 2,3-Dihydroisothiazol-4-yl, 2,3-Dihydroisothiazol-5-yl, Δ³-1,2-Dithiol-3-yl, Δ³-1,2-Dithiol-4-yl, Δ³-1,2-Dithiol-5-yl, 4,5-Dihydro-1H-imidazol-4-yl, 4,5-Dihydro-1H-imidazol-5-yl, 2,5-Dihydro-1H-imidazol-4-yl, 2,5-Dihydro-1H-imidazol-5-yl, 2,3-Dihydro-1H-imidazol-4-yl, 4,5-Dihydrooxazol-4-yl, 4,5-Dihydrooxazol-5-yl, 2,5-Dihydrooxazol-4-yl, 2,5-Dihydrooxazol-5-yl, 2,3-Dihydrooxazol-4-yl, 2,3-Dihydrooxazol-5-yl, 4,5-Dihydrothiazol-4-yl, 4,5-Dihydrothiazol-5-yl, 2,5-Dihydrothiazol-4-yl, 2,5-Dihydrothiazol-5-yl, 2,3-Dihydrothiazol-4-yl, 2,3-Dihydrothiazol-5-yl, 1,3-Dioxol-4-yl, 1,3-Dithiol-4-yl, 1,3-Oxathiol-4-yl, 1,3-Oxathiol-5-yl, 1,3-Dioxan-4-yl, 1,3-Dioxan-5-yl, 1,4-Dioxan-2-yl, 1,3-Dithian-4-yl, 1,3-Dithian-5-yl, 1,4-Dithian-2-yl, 1,3-Oxathian-4-yl, 1,3-Oxathian-5-yl, 1,3-Oxathian-6-yl, 1,4-Oxathian-2-yl, 1,4-Oxathian-3-yl, 1,2-Dithian-3-yl, 1,2-Dithian-4-yl, Hexahydropyrimidin-4-yl, Hexahydropyrimidin-5-yl, Hexahydropyrazin-2-yl, Hexahydropyridazin-3-yl, Hexahydropyridazin-4-yl, Tetrahydro-1,3-oxazin-4-yl, Tetrahydro-1,3-oxazin-5-yl, Tetrahydro-1,3-oxazin-6-yl, Tetrahydro-1,3-thiazin-4-yl, Tetrahydro-1,3-thiazin-5-yl, Tetrahydro-1,3-thiazin-6-yl, Tetrahydro-1,4-thiazin-2-yl, Tetrahydro-1,4-thiazin-3-yl, Tetrahydro-1,4-oxazin-2-yl, Tetrahydro-1,4-oxazin-3-yl, Tetrahydro-1,2-oxazin-3-yl, Tetrahydro-1,2-oxazin-4-yl, Tetrahydro-1,2-oxazin-5-yl, Tetrahydro-1,2-oxazin-6-yl, 2H-5,6-Dihydro-1,2-oxazin-3-yl, 2H-5,6-Dihydro-1,2-oxazin-4-yl, 2H-5,6-Dihydro-1,2-oxazin-5-yl, 2H-5,6-Dihydro-1,2-oxazin-6-yl, 2H-5,6-Dihydro-1,2-thiazin-3-yl, 2H-5,6-Dihydro-1,2-thiazin-4-yl, 2H-5,6-Dihydro-1,2-thiazin-5-yl, 2H-5,6-Dihydro-1,2-thiazin-6-yl, 4H-5,6-Dihydro-1,2-oxazin-3-yl, 4H-5,6-Dihydro-1,2-oxazin-4-yl, 4H-5,6-Dihydro-1,2-oxazin-5-yl, 4H-5,6-Dihydro-1,2-oxazin-6-yl, 4H-5,6-Dihydro-1,2-thiazin-3-yl, 4H-5,6-Dihydro-1,2-thiazin-4-yl, 4H-5,6-Dihydro-1,2-thiazin-5-yl, 4H-5,6-Dihydro-1,2-thiazin-6-yl, 2H-3,6-Dihydro-1,2-oxazin-3-yl, 2H-3,6-Dihydro-1,2-oxazin-4-yl, 2H-3,6-Dihydro-1,2-oxazin-5-yl, 2H-3,6-Dihydro-1,2-oxazin-6-yl, 2H-3,6-Dihydro-1,2-thiazin-3-yl, 2H-3,6-Dihydro-1,2-thiazin-4-yl, 2H-3,6-Dihydro-1,2-thiazin-5-yl, 2H-3,6-Dihydro-1,2-thiazin-6-yl, 2H-3,4-Dihydro-1,2-oxazin-3-yl, 2H-3,4-Dihydro-1,2-oxazin-4-yl, 2H-3,4-Dihydro-1,2-oxazin-5-yl, 2H-3,4-Dihydro-1,2-oxazin-6-yl, 2H-3,4-Dihydro-1,2-thiazin-3-yl, 2H-3,4-Dihydro-1,2-thiazin-4-yl, 2H-3,4-Dihydro-1,2-thiazin-5-yl, 2H-3,4-Dihydro-1,2-thiazin-6-yl, 2,3,4,5-Tetrahydropyridazin-3-yl, 2,3,4,5-Tetrahydropyridazin-4-yl, 2,3,4,5-Tetrahydropyridazin-5-yl, 2,3,4,5-Tetrahydropyridazin-6-yl, 3,4,5,6-Tetrahydropyridazin-3-yl, 3,4,5,6-Tetrahydropyridazin-4-yl, 1,2,5,6-Tetrahydropyridazin-3-yl, 1,2,5,6-Tetrahydropyridazin-4-yl, 1,2,5,6-Tetrahydropyridazin-5-yl, 1,2,5,6-Tetrahydropyridazin-6-yl, 1,2,3,6-Tetrahydropyridazin-3-yl, 1,2,3,6-Tetrahydropyridazin-4-yl, 4H-5,6-Dihydro-1,3-oxazin-4-yl, 4H-5,6-Dihydro-1,3-oxazin-5-yl, 4H-5,6-Dihydro-1,3-oxazin-6-yl, 4H-5,6-Dihydro-1,3-thiazin-4-yl, 4H-5,6-Dihydro-1,3-thiazin-5-yl, 4H-5,6-Dihydro-1,3-thiazin-6-yl, 3,4,5,6-Tetrahydropyrimidin-4-yl, 3,4,5,6-Tetrahydropyrimidin-5-yl, 3,4,5,6-Tetrahydropyrimidin-6-yl, 1,2,3,4-Tetrahydropyrazin-2-yl, 1,2,3,4-Tetrahydropyrazin-5-yl, 1,2,3,4-Tetrahydropyrimidin-4-yl, 1,2,3,4-Tetrahydropyrimidin-5-yl, 1,2,3,4-Tetrahydropyrimidin-6-yl, 2,3-Dihydro-1,4-thiazin-2-yl, 2,3-Dihydro-1,4-thiazin-3-yl, 2,3-Dihydro-1,4-thiazin-5-yl, 2,3-Dihydro-1,4-thiazin-6-yl, 2H-1,2-Oxazin-3-yl, 2H-1,2-Oxazin-4-yl, 2H-1,2-Oxazin-5-yl, 2H-1,2-Oxazin-6-yl, 2H-1,2-Thiazin-3-yl, 2H-1,2-Thiazin-4-yl, 2H-1,2-Thiazin-5-yl, 2H-1,2-Thiazin-6-yl, 4H-1,2-Oxazin-3-yl, 4H-1,2-Oxazin-4-yl, 4H-1,2-Oxazin-5-yl, 4H-1,2-Oxazin-6-yl, 4H-1,2-Thiazin-3-yl, 4H-1,2-Thiazin-4-yl, 4H-1,2-Thiazin-5-yl, 4H-1,2-Thiazin-6-yl, 6H-1,2-Oxazin-3-yl, 6H-1,2-Oxazin-4-yl, 6H-1,2-Oxazin-5-yl, 6H-1,2-Oxazin-6-yl, 6H-1,2-Thiazin-3-yl, 6H-1,2-Thiazin-4-yl, 6H-1,2-Thiazin-5-yl, 6H-1,2-Thiazin-6-yl, 2H-1,3-Oxazin-4-yl, 2H-1,3-Oxazin-5-yl, 2H-1,3-Oxazin-6-yl, 2H-1,3-Thiazin-4-yl, 2H-1,3-Thiazin-5-yl, 2H-1,3-Thiazin-6-yl, 4H-1,3-Oxazin-4-yl, 4H-1,3-Oxazin-5-yl, 4H-1,3-Oxazin-6-yl, 4H-1,3-Thiazin-4-yl, 4H-1,3-Thiazin-5-yl, 4H-1,3-Thiazin-6-yl, 6H-1,3-Oxazin-4-yl, 6H-1,3-Oxazin-5-yl, 6H-1,3-Oxazin-6-yl, 6H-1,3-Oxazin-4-yl, 6H-1,3-Oxazin-5-yl, 6H-1,3-Thiazin-6-yl, 2H-1,4-Oxazin-2-yl, 2H-1,4-Oxazin-3-yl, 2H-1,4-Oxazin-5-yl, 2H-1,4-Oxazin-6-yl, 2H-1,4-Thiazin-2-yl, 2H-1,4-Thiazin-3-yl, 2H-1,4-Thiazin-5-yl, 2H-1,4-Thiazin-6-yl, 4H-1,4-Oxazin-2-yl, 4H-1,4-Oxazin-3-yl, 4H-1,4-Thiazin-2-yl, 4H-1,4-Thiazin-3-yl, 1,4-Dihydropyridazin-3-yl, 1,4-Dihydropyridazin-4-yl, 1,4-Dihydropyridazin-5-yl, 1,4-Dihydropyridazin-6-yl, 1,4-Dihydropyrazin-2-yl, 1,2-Dihydropyrazin-2-yl, 1,2-Dihydropyrazin-3-yl, 1,2-Dihydropyrazin-5-yl, 1,2-Dihydropyrazin-6-yl, 1,4-Dihydropyrimidin-4-yl, 1,4-Dihydropyrimidin-5-yl, 1,4-Dihydropyrimidin-6-yl, 3,4-Dihydropyrimidin-4-yl, 3,4-Dihydropyrimidin-5-yl oder 3,4-Dihydropyrimidin-6-yl, wobei die voranstehend genannten Fünfringheterocyclen einen bis drei an Kohlenstoff gebundene Reste, ausgewählt aus der Gruppe R⁶, R⁷ und R⁸ und ggf. jeder Ringstickstoff unabhängig voneinander einen Rest R¹⁰ und wobei die voranstehenden Sechsringheterocyclen einen bis vier an Kohlenstoff gebundene Reste, ausgewählt aus der Gruppe R⁶, R⁷, R⁸ und R⁹, und ggf. jeder Ringstickstoff unabhängig voneinander einen Rest R¹⁰, tragen können und/oder wobei ggf. der Schwefel der genannten Heterocyclen zu S=O oder S(=O)₂ oxidiert sein kann.

Ganz außerordentlich bevorzugt sind ferner substituierte Carbamoyltriazole der Formel I, wobei A für Tetrahydroimidazol-2-yl, Tetrahydrooxazol-2-yl, Tetrahydrothiazol-2-yl, 1,3-Oxathiolan-2-yl, 1,3-Dithiolan-2-yl, 4,5-Dihydro-1H-imidazol-2-yl, 2,5-Dihydro-1H-imidazol-2-yl, 2,3-Dihydro-1H-imidazol-2-yl, 4,5-Dihydrooxazol-2-yl, 2,5-Dihydrooxazol-2-yl, 2,3-Dihydrooxazol-2-yl, 4,5-Dihydrothiazol-2-yl, 2,5-Dihydrothiazol-2-yl, 2,3-Dihydrothiazol-2-yl, 1,3-Dithiol-2-yl, 1,3-Oxathiol-2-yl, 1,3-Dithian-2-yl, 1,3-Oxathian-2-yl, Hexahydropyrimidin-2-yl, Tetrahydro-1,3-oxazin-2-yl, Tetrahydro-1,3-thiazin-2-yl, 4H-5,6-Dihydro-1,3-oxazin-2-yl, 4H-5,6-Dihydro-1,3-thiazin-2-yl, 3,4,5,6-Tetrahydropyrimidin-2-yl, 1,2,3,4-Tetrahydropyrimidin-2-yl, 2H-1,3-Oxazin-2-yl, 2H-1,3-Thiazin-2-yl, 4H-1,3-Oxazin-2-yl, 4H-1,3-Thiazin-2-yl, 6H-1,3-Oxazin-2-yl, 6H-1,3-Thiazin-2-yl, 2,3-Dihydropyrimidin-2-yl, 2,5-Dihydropyrimidin-2-yl, 3,4-Dihydropyrimidin-2-yl oder 4,5-Dihydropyrimidin-2-yl, steht, wobei die voranstehend genannten Fünfringheterocyclen einen bis drei an Kohlenstoff gebundene Reste, ausgewählt aus der Gruppe R⁶, R⁷ und R⁸ und ggf. jeder Ringstickstoff unabhängig voneinander einen Rest R¹⁰ und wobei die voranstehend Sechsringheterocyclen einen bis vier an Kohlenstoff gebundene Reste, ausgewählt aus der Gruppe R⁶, R⁷, R⁸ und R⁹, und ggf. jeder Ringstickstoff unabhängig voneinander einen Rest R¹⁰ tragen können und/oder wobei ggf. der Schwefel der genannten Heterocyclen zu S=O oder S(=O)₂ oxidiert sein kann.

Ganz außerordentlich bevorzugt sind ferner substituierte Carbamoyltriazole der Formel I, wobei A für 1,3-Dioxolan-2-yl, 1,3-Dioxol-2-yl oder 1,3-Dioxan-2-yl steht, wobei die voranstehend genannten Fünfringheterocyclen einen bis drei an Kohlenstoff gebundene Reste, ausgewählt aus der Gruppe R⁶, R⁷ und R⁸, und wobei die voranstehenden Sechsringheterocyclen einen bis vier an Kohlenstoff gebundene Reste, ausgewählt aus der Gruppe R⁶, R⁷, R⁸ und R⁹ tragen.

Außerordentlich bevorzugt sind ferner substituierte Carbamoyltriazole der Formel I, wobei die Variablen folgende Bedeutung haben:
- R¹, R²: Ethyl
- R³: Wasserstoff
- R⁴: Wasserstoff
- R⁵: Wasserstoff, Methyl

- A: Tetrahydrofuran-2-yl, Tetrahydrofuran-3-yl, Tetrahydrothien-3-yl, Tetrahydroisoxazol-5-yl, 1,3-Dioxolan-4-yl, 4,5-Dihydroisoxazol-5-yl, Tetrahydropyran-2-yl, Tetrahydropyran-3-yl, Tetrahydropyran-4-yl, Tetrahydrothiopyran-3-yl, Tetrahydrothiopyran-4-yl, 2H-3,4-Dihydropyran-4-yl oder 2H-5,6-Dihydrothiopyran-3-yl, wobei die voranstehend genannten Heterocyclen unsubstituiert oder durch ein oder zwei Reste, ausgewählt aus folgender Gruppe: C₁-C₆-Alkyl, C₁-C₆-Alkoxy, Phenyl oder Benzyl, das seinerseits partiell oder vollständig halogeniert sein kann, und einen bis drei der folgenden Gruppe tragen kann: Nitro Cyano, C₁-C₄-Alkyl, C₁-C₄-Halogenalkyl, C₁-C₄-Alkoxy, C₁-C₄-Halogenalkoxy und C₁-C₄-Alkoxycarbonyl, substituiert sein können und wobei ggf. der Schwefel der Heterocyclen zu S=O oder S(=O)₂ oxidiert ist;
1,3-Dioxan-2-yl, das durch ein oder zwei C₁-C₆-Alkyl-Reste substituiert ist;
- n: 0 oder 2

Die substituierten Carbamoyltriazole der Formel I sind auf verschiedene Weise erhältlich, beispielsweise nach einem der folgenden Verfahren:

### Verfahren A

Umsetzung eines Triazols der Formel II, mit einem Carbamidsäurehalogenid der Formel III

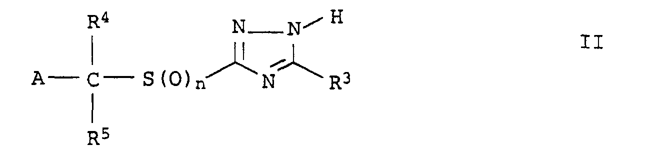

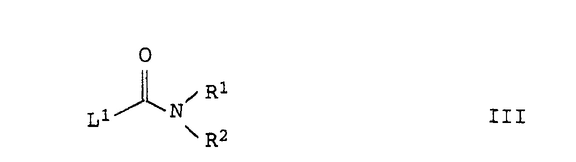

L¹ steht für Halogen, vorzugsweise Chlor.

In der Regel wird die Umsetzung in einem inerten organischen Lösungsmittel vorgenommen. Es werden beispielsweise aromatische Kohlenwasserstoffe wie Benzol oder Toluol, halogenierte Kohlenwasserstoffe wie Methylenchlorid, 1,2-Dichlorethan oder Chlorbenzol, Ether wie Diethylether, Methyl-tert.-butylether, Tetrahydrofuran oder Dioxan, Ketone wie Aceton oder Methylethylketon, oder polare Lösungsmittel wie Dimethylformamid, Diethylformamid oder Dimethylsulfoxid oder Gemische hiervon verwendet.

Es kann von Vorteil sein, in Gegenwart einer Base zu arbeiten. Als Basen kommen u.a. Alkalimetallalkoholate, insbesondere Natriummethanolat oder Natriumethanolat, Alkalimetallhydroxide, insbesondere Natriumhydroxid oder Kaliumhydroxid, Alkalimetallcarbonate, insbesondere Natriumcarbonat oder Kaliumcarbonat, Metallhydride wie Natriumhydrid, organische Basen, z.B. Amine wie Triethylamin, Pyridin oder N,N-Dimethylaminopyridin oder Gemische hiervon in Betracht.

Die Ausgangsverbindungen werden in der Regel im äquimolaren Verhältnis eingesetzt. Es kann aber auch von Vorteil sein, die eine oder andere Komponente im Überschuß einzusetzen.

Die Menge der Base liegt vorzugsweise bei der 0,5- bis zweifachen molaren Menge, bezogen auf die Ausgangsverbindungen.

In der Regel liegt die Reaktionstemperatur im Bereich von 0°C bis zur Höhe der Siedetemperatur des Reaktionsgemisches.

### Verfahren B

Umsetzung von substituierten Carbamoyltriazolen der Formel I mit n=0, 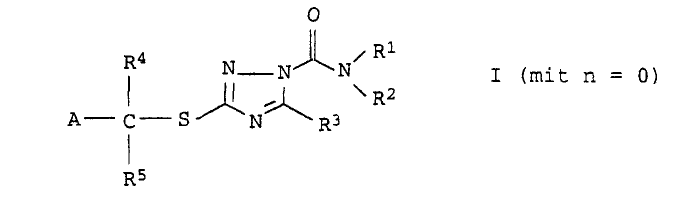 mit Oxidationsmitteln zu substituierten Carbamoyltriazolen der Formel I mit n=1 oder 2.

Als Oxidationsmittel können u.a. organische Peroxide wie m-Chlorperbenzoesäure, Essigsäureperoxid oder Trifluoressigsäureperoxid, anorganische Peroxide wie Wasserstoffperoxid oder Natriumperjodat verwendet werden.

Es kann auch von Vorteil sein, Katalysatoren wie Wolframate zuzusetzen.

Als Lösungsmittel kommen in Abstimmung mit dem Oxidationsmittel aromatische Kohlenwasserstoffe wie Benzol oder Toluol, halogenierte Kohlenwasserstoffe wie Methylenchlorid, Chloroform, 1,2-Dichlorethan oder Chlorbenzol, organische Säuren wie Essigsäure, Propionsäure, Trifluoressigsäure oder Gemische hiervon in Betracht.

Das Oxidationsmittel kann in äquimolarer Menge bezüglich des Edukts eingesetzt werden; es kann aber auch von Vorteil sein, einen Überschuß von bis zu 10 Moläquivalenten einzusetzen.

Der Oxidationskatalysator wird in der Regel in einem Bereich von 1 bis 20 mol-%, bezogen auf das Edukt, eingesetzt.

In der Regel liegt die Reaktionstemperatur in dem Bereich von 0°C bis zur Höhe der Siedetemperatur des Reaktionsgemisches.

Die Triazole der Formel II mit n = 2 sind neu, wobei die weitere Variablen die voranstehende Bedeutung haben.

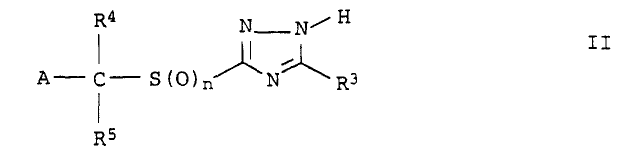

Die besonders bevorzugten Ausführungsformen der Triazole der Formel II in Bezug auf die Variablen R¹ bis R⁵ entsprechend denen der substituierten Carbamoyltriazole der Formel I.

Die Darstellung der Verbindungen der Formel II mit n=0 erfolgt beispielsweise durch Umsetzung eines Mercaptotriazols der Formel IV 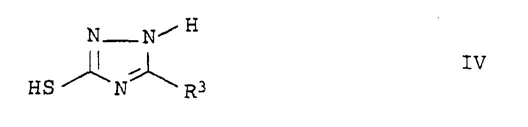 mit einer Verbindung der Formel V, 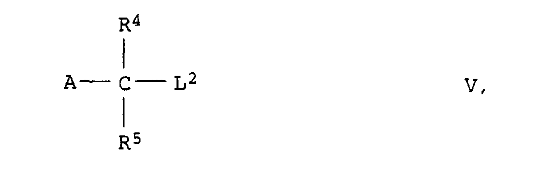 wobei L² für eine nucleophil verdrängbare Abgangsgruppe wie Halogen, z.B. Chlor oder Brom, C₁-C₆-Alkylsulfonyloxy, z.B. Mesylat, C₁-C₆-Halogenalkylsulfonyloxy, z.B. Triflat oder Arylsulfonyloxy, z.B. Tosylat, steht.

Die Synthese dieser Verbindungen erfolgt in Analogie zu den unter Verfahren A beschriebenen Bedingungen.

Die Synthese der Verbindungen der Formel II mit n = 1 oder 2 erfolgt durch Oxidation von Verbindungen der Formel II mit n = 0 gemäß den unter Verfahren B beschriebenen Bedingungen.

Die Aufarbeitung der Reaktionsgemische erfolgt in der Regel nach an sich bekannten Methoden, beispielsweise durch Entfernen des Lösungsmittels, Verteilen des Rückstandes in einem Gemisch aus Wasser und organischem Lösungsmittel und Aufarbeitung der organischen Phase auf das Produkt hin.

### Herstellbeispiele

### Beispiel 1: 1-Diethylcarbamoyl-3-(tetrahydropyran-2-yl)methylsulfonyl-1,2,4-triazol (Verbindung 2.02)

### a) (Tetrahydropyran-2-yl-methyl)-methansulfonat

In 200 ml Toluol wurden 23,2 g (200 mmol) Tetrahydropyran-2-yl-methanol mit 27,5 g (240 mmol) Methansulfonsäurechlorid versetzt. Anschließend wurden bei 10°C 24,3 g (240 mmol) Triethylamin zugetropft und 2 Stunden bei Raumtemperatur gerührt. Dann wurde mit 200 ml Methyl-tert.-Butylether und 200 ml Wasser versetzt, die organische Phase abgetrennt, diese mit 200 ml Wasser gewaschen und getrocknet. Nach Entfernen des Lösungsmittels am Vakuum erhielt man 33,7 g (87 % d.Th.) eines hellen Öls.
(¹H-NMR (CDCl₃; δ in ppm): 4,20 (d,2H); 4,05 (dd,1H); 3,60 (m, 1H); 3,45 (m, 1H); 3,1 (s,3H); 1,95 (m, 1H); 1,45 (m,5H))

### b) 3-(Tetrahydropyran-2-yl)methylthio-1,2,4-triazol

In 200 ml Methanol wurden 9,7 g (173 mmol) Kaliumhydroxid und 17,5 g (173 mmol) 3-Mercapto-1,2,4-triazol gelöst und eine Stunde unter Rückfluß erhitzt. Nach Abdestillieren des Lösungsmittels am Vakuum wurde der Rückstand durch azeotrope Destillation mit Toluol getrocknet und dann in 300 ml Methanol aufgenommen. Nun wurden 33,6 g (173 mmol) (Tetrahydropyran-2-yl-methyl)-methansulfonat (Stufe a) zugesetzt und 24 Stunden bei Raumtemperatur gerührt. Anschließend wurde das Reaktionsgemisch mit 200 ml Wasser und 200 ml Methyl-tert. -butylether versetzt, die organische Phase abgetrennt, mit Wasser gewaschen und getrocknet. Nach Einengen und Chromatographie des Rückstandes an Kieselgel erhielt man 18,2 g (53 % d.Th.) eines gelben Öls.
(¹H-NMR (CDCl₃: δ in ppm): 8,05 (s,1H); 4,15 (d,1H); 3,65 (m, 1H); 3,50 (m,1H); 3,25-3,10 (m,2H); 1,85 (m, 1H); 1,75-1,25 (m,6H))

### c) 1-Diethylcarbamoyl-3-(tetrahydropyran-2-yl)methylthio-1,2,4-triazol (Verbindung 2.01)

Zu einer Lösung aus 200 ml absolutem Tetrahydrofuran und 17,0 g (85 mmol) 3-(Tetrahydropyran-2-yl)methylthio-1,2,4-triazol (Stufe b) wurden 11,6 g (85 mmol) Diethylcarbamidsäurechlorid und eine Spatelspitze Dimethylaminopyridin gegeben. Anschließend wurden 9,5 g (94 mmol) Triethylamin zugetropft und 2 Stunden am Rückfluß erhitzt. Nach Abdestillieren des Lösungsmittels wurde der Rückstand durch Chromatographie an Kieselgel gereinigt. Man erhielt 11,2 g (44 % d.Th.) eines zähen, klaren Öls.
(¹H-NMR (CDCl₃; δ in ppm): 8,75 (s,1H); 4,00 (d,1H); 3,70-3,50 (bm,5H); 3,40 (dt,1H); 3,25 (d,2H); 1,85 (d,1H); 1,80 (d,1H); 1,65-1,45 (m,4H); 1,30 (t,6H))

### d) 1-Diethylcarbamoyl-3- (tetrahydropyran-2-yl)methylsulfonyl-1,2,4-triazol

In 100 ml Eisessig wurden 5,0 g (17 mmol) 1-Diethylcarbamoyl-3-(tetrahydropyran-2-yl)methylthio-1-2,4-triazol (Stufe c) gelöst und mit einer Spatelspitze Natriumwolframat versetzt. Bei 50°C wurden dann 4,8 g (42 mmol) Wasserstoffperoxid (als 30%ige Lösung in Wasser) zugetropft und 2 Stunden am Rückfluß erhitzt. Nach dem Abkühlen wurde das Reaktionsgemisch mit 100 ml Wasser und 100 ml Dichlormethan versetzt, die organische Phase mit Natriumdithionit-Lösung, anschließend mit Natriumhydrogencarbonat-Lösung gewaschen und getrocknet. Nach Einengen wurde der Rückstand mit wenig Diethylether digeriert und abgesaugt. Man erhielt 3,0 g (50 % d.Th.) eines weißen, kristallinen Pulvers (Schmelzpunkt 122°C).
(¹H-NMR (CDCl₃; δ in ppm): 8,85 (s, 1H); 3,95 (m,1H); 3,70 (m,2H); 3,70-3,50 (bm,4H)); 3,35 (m,2H); 1,85 (m,1H); 1,70 (dd,1H); 1,55-1,40 (m,4H); 1,30 (d,6H))

In Tabelle 2 sind neben dem voranstehend beschriebenen substituierten Carbamoyltriazol der Formel I weitere aufgeführt, die in analoger Weise oder auf an sich bekannte Art und Weise hergestellt wurden oder herstellbar sind:

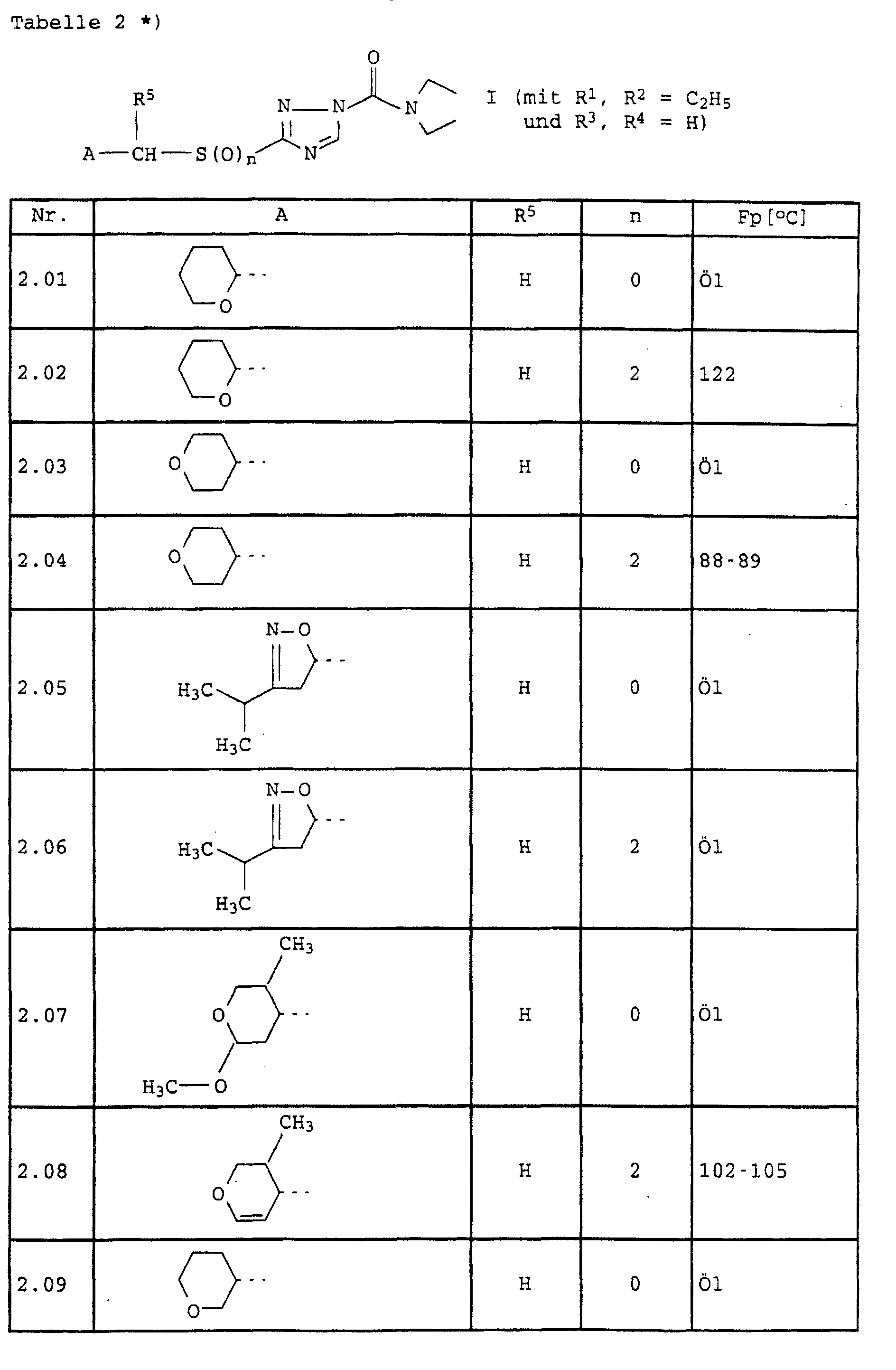

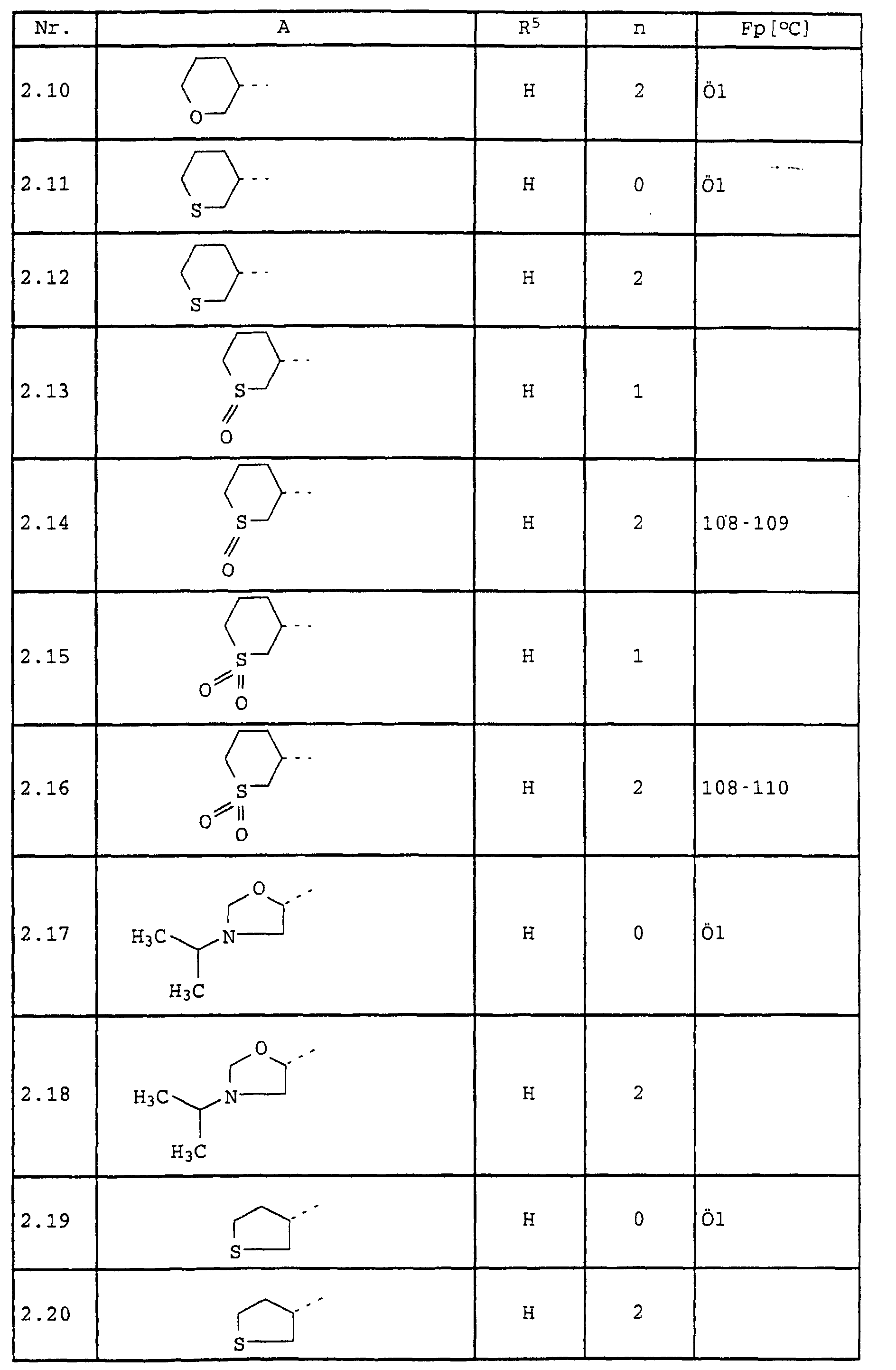

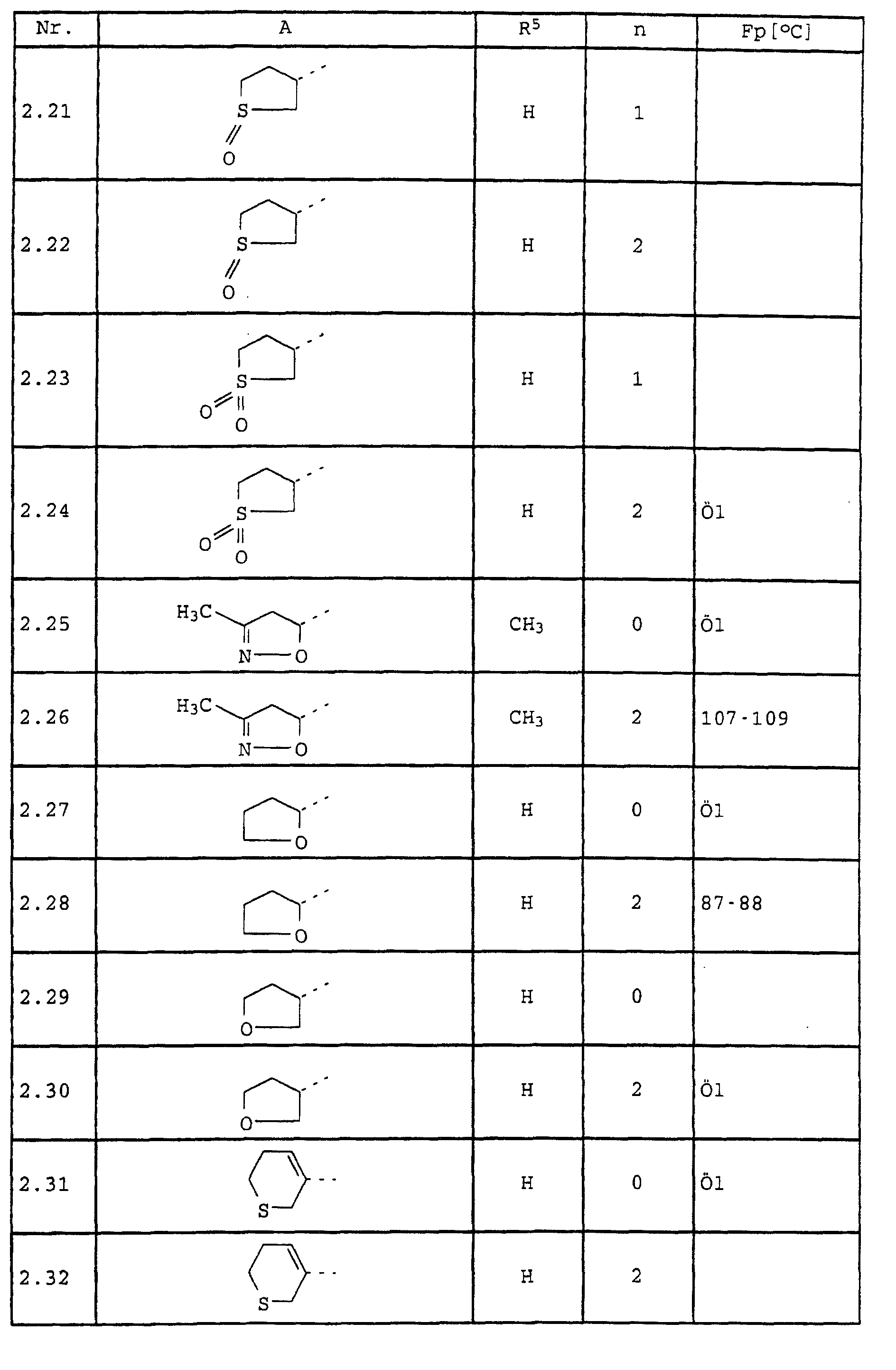

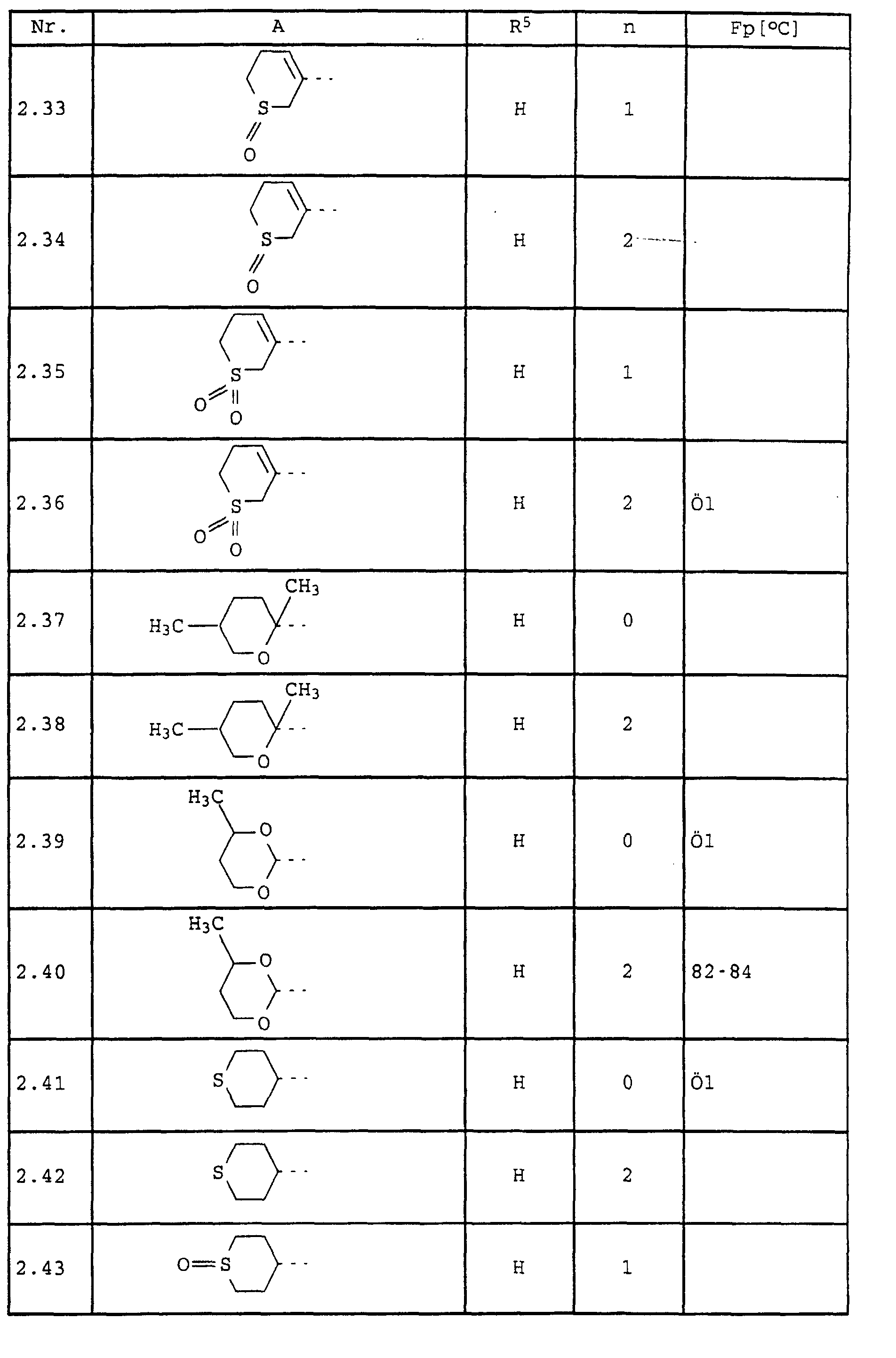

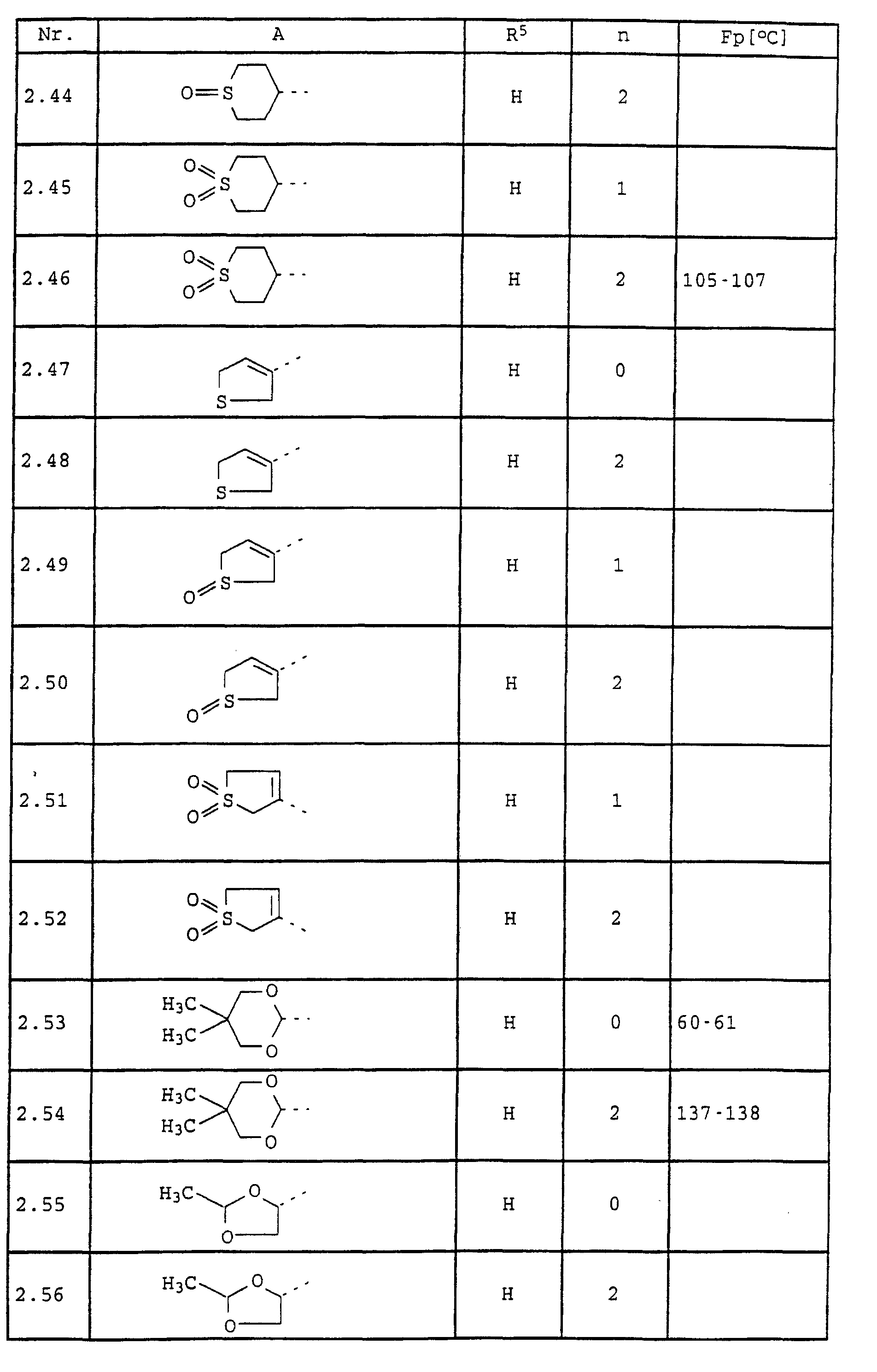

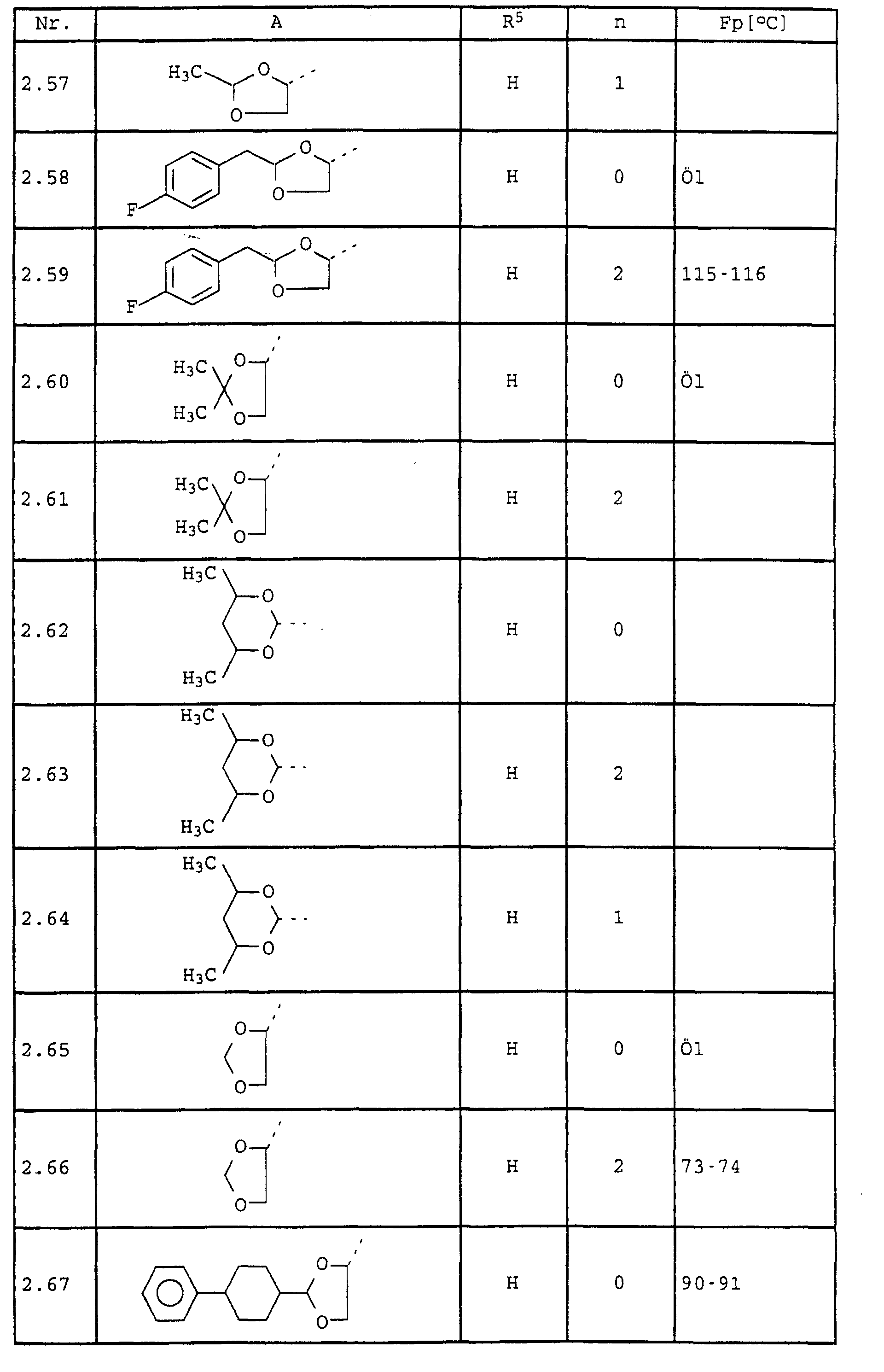

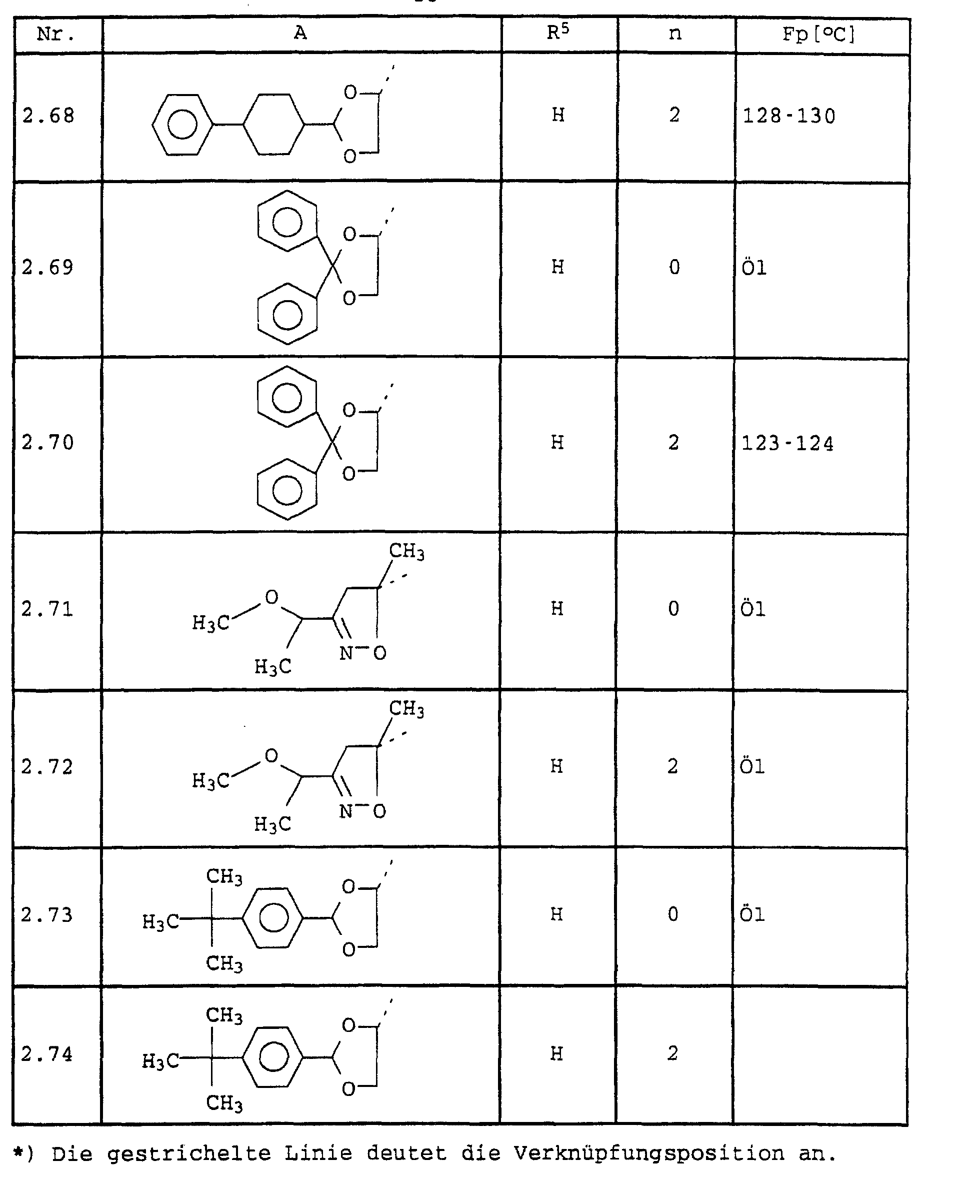

In Tabelle 3 sind neben den voranstehend beschriebenen Triazolen der Formel II weitere aufgeführt, die in analoger Weise oder auf an sich bekannte Art und Weise hergestellt wurden oder herstellbar sind:

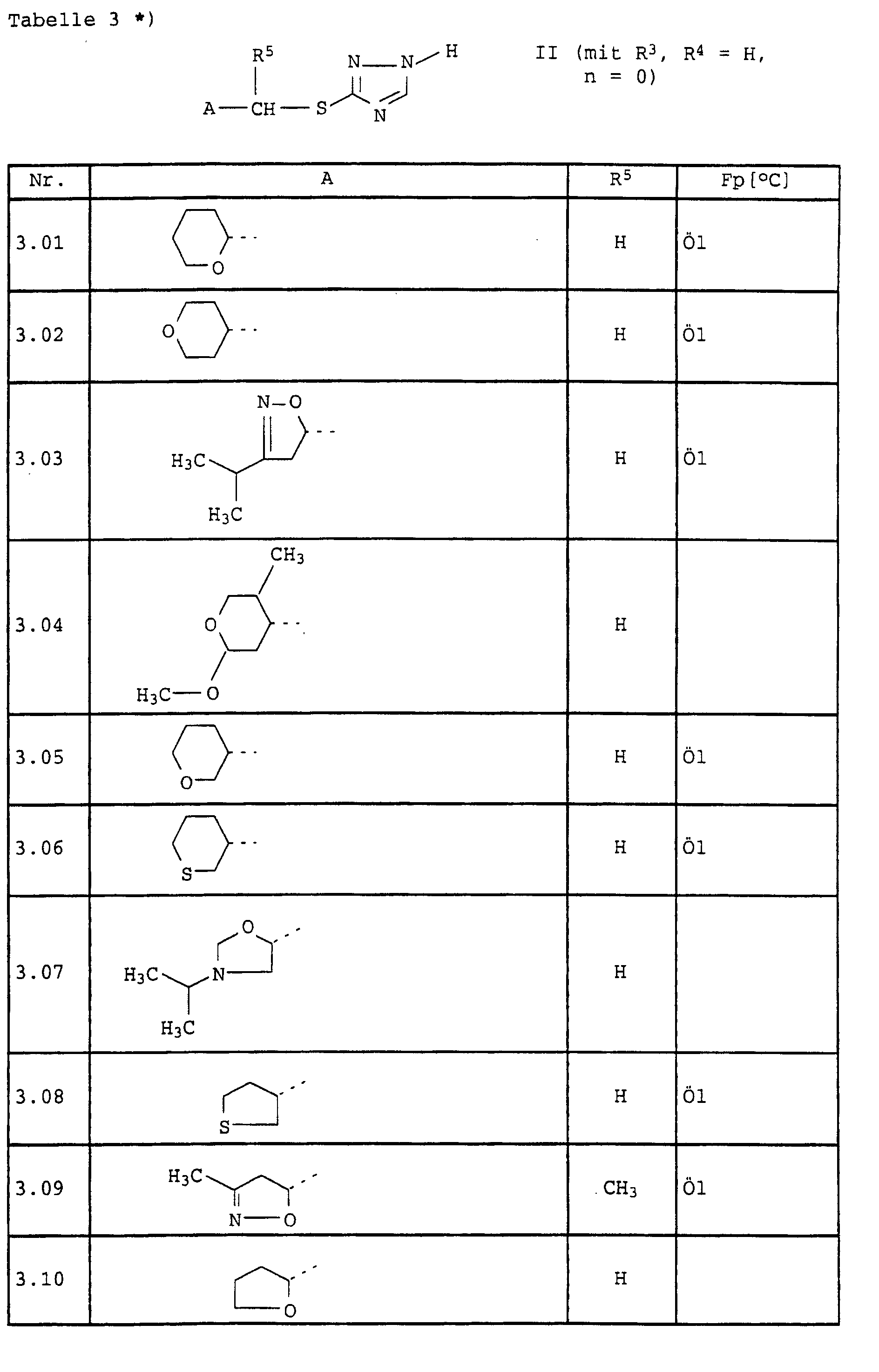

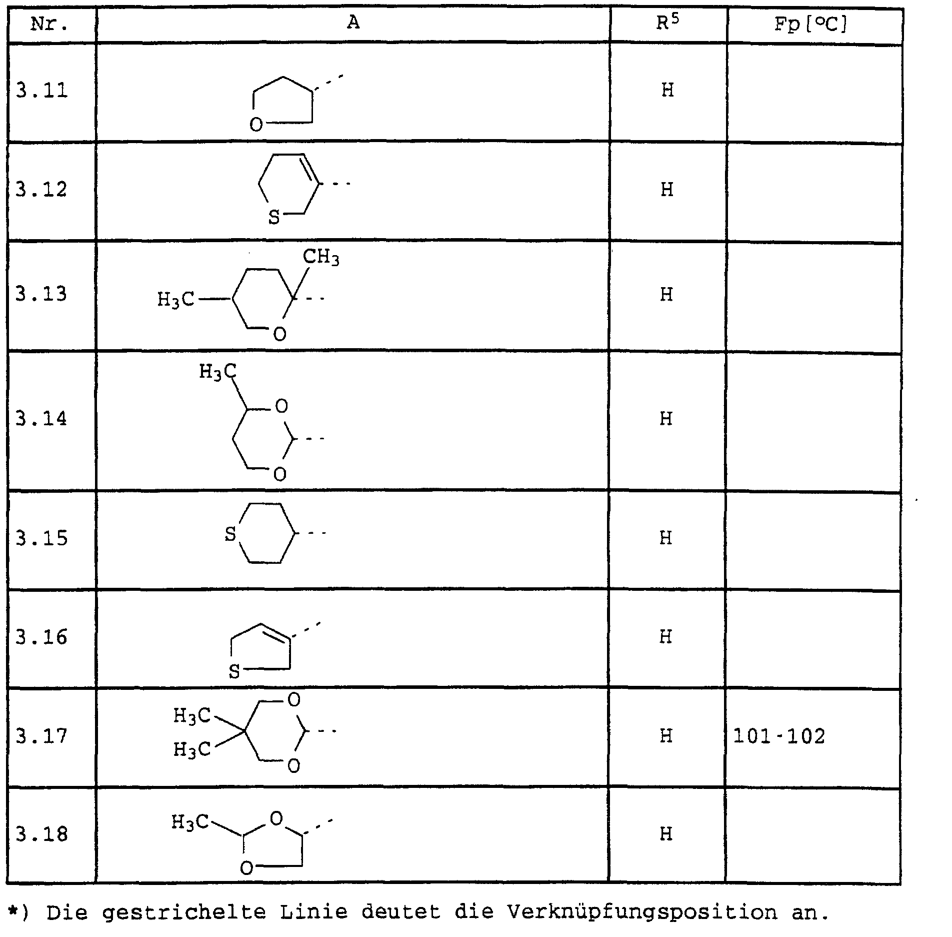

Die Verbindungen der Formel I und deren landwirtschaftlich brauchbaren Salze eignen sich - sowohl als Isomerengemische als auch in Form der reinen Isomeren - als Herbizide und/oder als Bioregulatoren. Die Verbindungen der Formel I enthaltenden herbiziden Mittel bekämpfen Pflanzenwuchs auf Nichtkulturflächen sehr gut, besonders bei hohen Aufwandmengen. In Kulturen wie Weizen, Reis, Mais, Soja und Baumwolle wirken sie gegen Unkräuter und Schadgräser, ohne die Kulturpflanzen nennenswert zu schädigen. Dieser Effekt tritt vor allem bei niedrigen Aufwandmengen auf.

In Abhängigkeit von der jeweiligen Applikationsmethode können die Verbindungen der Formel I bzw. sie enthaltenden herbiziden Mittel noch in einer weiteren Zahl von Kulturpflanzen zur Beseitigung unerwünschter Pflanzen eingesetzt werden. In Betracht kommen beispielsweise folgende Kulturen:

Allium cepa, Ananas comosus, Arachis hypogaea, Asparagus officinalis, Beta vulgaris spec. altissima, Beta vulgaris spec. rapa, Brassica napus var. napus, Brassica napus var. napobrassica, Brassica rapa var. silvestris, Camellia sinensis, Carthamus tinctorius, Carya illinoinensis, Citrus limon, Citrus sinensis, Coffea arabica (Coffea canephora, Coffea liberica), Cucumis sativus, Cynodon dactylon, Daucus carota, Elaeis guineensis, Fragaria vesca, Glycine max, Gossypium hirsutum, (Gossypium arboreum, Gossypium herbaceum, Gossypium vitifolium), Helianthus annuus, Hevea brasiliensis, Hordeum vulgare, Humulus lupulus, Ipomoea batatas, Juglans regia, Lens culinaris, Linum usitatissimum, Lycopersicon lycopersicum, Malus spec., Manihot esculenta, Medicago sativa, Musa spec., Nicotiana tabacum (N.rustica), Olea europaea, Oryza sativa , Phaseolus lunatus, Phaseolus vulgaris, Picea abies, Pinus spec., Pisum sativum, Prunus avium, Prunus persica, Pyrus communis, Ribes sylvestre, Ricinus communis, Saccharum officinarum, Secale cereale, Solanum tuberosum, Sorghum bicolor (s. vulgare), Theobroma cacao, Trifolium pratense, Triticum aestivum, Triticum durum, Vicia faba, Vitis vinifera und Zea mays.

Darüber hinaus können die Verbindungen der Formel I auch in Kulturen, die durch Züchtung einschließlich gentechnischer Methoden gegen die Wirkung von Herbiziden tolerant sind, verwandt werden.

Die Verbindungen der Formel I bzw. die sie enthaltenden herbiziden Mittel können beispielsweise in Form von direkt versprühbaren wäßrigen Lösungen, Pulvern, Suspensionen, auch hochprozentigen wäßrigen, öligen oder sonstigen Suspensionen oder Dispersionen, Emulsionen, Öldispersionen, Pasten, Stäubemitteln, Streumitteln oder Granulaten durch Versprühen, Vernebeln, Verstäuben, Verstreuen oder Gießen angewendet werden. Die Anwendungsformen richten sich nach den Verwendungszwecken; sie sollten in jedem Fall möglichst die feinste Verteilung der erfindungsgemäßen Wirkstoffe gewährleisten.

Als inerte Hilfsstoffe kommen im Wesentlichen in Betracht: Mineralölfraktionen von mittlerem bis hohem Siedepunkt wie Kerosin und Dieselöl, ferner Kohlenteeröle sowie Öle pflanzlichen oder tierischen Ursprungs, aliphatische, cyclische und aromatische Kohlenwasserstoffe, z.B. Paraffine, Tetrahydronaphthalin, alkylierte Naphthaline und deren Derivate, alkylierte Benzole und deren Derivate, Alkohole wie Methanol, Ethanol, Propanol, Butanol und Cyclohexanol, Ketone wie Cyclohexanon, stark polare Lösungsmittel, z.B. Amine wie N-Methylpyrrolidon und Wasser.

Wäßrige Anwendungsformen können aus Emulsionskonzentraten, Suspensionen, Pasten, netzbaren Pulvern oder wasserdispergierbaren Granulaten durch Zusatz von Wasser bereitet werden. Zur Herstellung von Emulsionen, Pasten oder Öldispersionen können die Substrate als solche oder in einem Öl oder Lösungsmittel gelöst, mittels Netz-, Haft-, Dispergier- oder Emulgiermittel in Wasser homogenisiert werden. Es können aber auch aus wirksamer Substanz, Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe (Adjuvantien) kommen die Alkali-, Erdalkali-, Ammoniumsalze von aromatischen Sulfonsäuren, z.B. Lignin-, Phenol-, Naphthalin- und Dibutylnaphthalinsulfonsäure, sowie von Fettsäuren, Alkyl- und Alkylarylsulfonaten, Alkyl-, Laurylether- und Fettalkoholsulfaten, sowie Salze sulfatierter Hexa-, Hepta- und Octadecanolen sowie von Fettalkoholglykolether, Kondensationsprodukte von sulfoniertem Naphthalin und seiner Derivate mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctyl-, Octyl- oder Nonylphenol, Alkylphenyl-, Tributylphenylpolyglykolether, Alkylarylpolyetheralkohole, Isotridecylalkohol, Fettalkoholethylenoxid-Kondensate, ethoxyliertes Rizinusöl, Polyoxyethylen- oder Polyoxypropylenalkylether, Laurylalkoholpolyglykoletheracetat, Sorbitester, Lignin-Sulfitablaugen oder Methylcellulose in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate können durch Bindung der Wirkstoffe an feste Trägerstoffe hergestellt werden. Feste Trägerstoffe sind Mineralerden wie Kieselsäuren, Kieselgele, Silikate, Talkum, Kaolin, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel wie Ammoniumsulfat, Ammoniumphosphat, Ammoniumnitrat, Harnstoffe und pflanzliche Produkte wie Getreidemehl, Baumrinden-, Holz- und Nußschalenmehl, Cellulosepulver oder andere feste Trägerstoffe.

Die Konzentrationen der Wirkstoffe der Formel I in den anwendungsfertigen Zubereitungen können in weiten Bereichen variiert werden. Im allgemeinen enthalten die Formulierungen etwa von 0,001 bis 98 Gew.-%, vorzugsweise 0,01 bis 95 Gew.-%, mindestens eines Wirkstoffs. Die Wirkstoffe werden dabei in einer Reinheit von 90% bis 100%, vorzugsweise 95% bis 100% (nach NMR-Spektrum) eingesetzt.

Die folgenden Formulierungsbeispiele verdeutlichen die Herstellung solcher Zubereitungen:
I. 20 Gewichtsteile der Verbindung Nr. 2.02 werden in einer Mischung gelöst, die aus 80 Gewichtsteilen alkyliertem Benzol, 10 Gewichtsteilen des Anlagerungsproduktes von 8 bis 10 Mol Ethylenoxid an 1 Mol Ölsäure-N-monoethanolamid, 5 Gewichtsteilen Calciumsalz der Dodecylbenzolsulfonsäure und 5 Gewichtsteilen des Anlagerungsproduktes von 40 Mol Ethylenoxid an 1 Mol Rizinusöl besteht. Durch Ausgießen und feines Verteilen der Lösung in 100000 Gewichtsteilen Wasser erhält man eine wäßrige Dispersion, die 0,02 Gew.-% des Wirkstoffs enthält.
II. 20 Gewichtsteile der Verbindung Nr. 2.04 werden in einer Mischung gelöst, die aus 40 Gewichtsteilen Cyclohexanon, 30 Gewichtsteilen Isobutanol, 20 Gewichtsteilen des Anlagerungsproduktes von 7 Mol Ethylenoxid an 1 Mol Isooctylphenol und 10 Gewichtsteilen des Anlagerungsproduktes von 40 Mol Ethylenoxid an 1 Mol Rizinusöl besteht. Durch Eingießen und feines Verteilen der Lösung in 100000 Gewichtsteilen Wasser erhält man eine wäßrige Dispersion, die 0,02 Gew.-% des Wirkstoffs enthält.
III. 20 Gewichtsteile des Wirkstoffs Nr. 2.06 werden in einer Mischung gelöst, die aus 25 Gewichtsteilen Cyclohexanon, 65 Gewichtsteilen einer Mineralölfraktion vom Siedepunkt 210 bis 280°C und 10 Gewichtsteilen des Anlagerungsproduktes von 40 Mol Ethylenoxid an 1 Mol Ricinusöl besteht. Durch Eingießen und feines Verteilen der Lösung in 100000 Gewichtsteilen Wasser erhält man eine wäßrige Dispersion, die 0,02 Gew.-% des Wirkstoffs enthält.
IV. 20 Gewichtsteile des Wirkstoffs Nr. 2.11 werden mit 3 Gewichtsteilen des Natriumsalzes der Diisobutylnaphthalin-α-sulfonsäure, 17 Gewichtsteilen des Natriumsalzes einer Ligninsulfonsäure aus einer Sulfit-Ablauge und 60 Gewichtsteilen pulverförmigem Kieselsäuregel gut vermischt und in einer Hammermühle vermahlen. Durch feines Verteilen der Mischung in 20000 Gewichtsteilen Wasser erhält man eine Spritzbrühe, die 0,1 Gew.-% des Wirkstoffs enthält.
V. 3 Gewichtsteile des Wirkstoffs Nr. 2.14 werden mit 97 Gewichtsteilen feinteiligem Kaolin vermischt. Man erhält auf diese Weise ein Stäubemittel, das 3 Gew.-% des Wirkstoffs enthält.
VI. 20 Gewichtsteile des Wirkstoffs Nr. 2.02 werden mit 2 Gewichtsteilen Calciumsalz der Dodecylbenzolsulfonsäure, 8 Gewichtsteilen Fettalkohol-polyglykolether, 2 Gewichtsteilen Natriumsalz eines Phenol-Harnstoff-Formaldehyd-Kondensates und 68 Gewichtsteilen eines paraffinischen Mineralöls innig vermischt. Man erhält eine stabile ölige Dispersion.
VII. 1 Gewichtsteil des Wirkstoffs Nr. 2.16 wird in einer Mischung gelöst, die aus 70 Gewichtsteilen Cyclohexanon, 20 Gewichtsteilen ethoxyliertem Isooctylphenol und 10 Gewichtsteilen ethoxyliertem Rizinusöl besteht. Man erhält ein stabiles Emulsionskonzentrat.
VIII. 1 Gewichtsteil des Wirkstoffs Nr. 2.26 wird in einer Mischung gelöst, die aus 80 Gewichtsteilen Cyclohexanon und 20 Gewichtsteilen Wettol® EM 31 (= nichtionischer Emulgator auf der Basis von ethoxyliertem Rizinusöl; BASF AG) besteht. Man erhält ein stabiles Emulsionskonzentrat.

Die Applikation der Wirkstoffe der Formel I bzw. der herbiziden Mittel kann im Vorauflauf- oder im Nachauflaufverfahren erfolgen. Sind die Wirkstoffe für gewisse Kulturpflanzen weniger verträglich, so können Ausbringungstechniken angewandt werden, bei welchen die herbiziden Mittel mit Hilfe der Spritzgeräte so gespritzt werden, daß die Blätter der empfindlichen Kulturpflanzen nach Möglichkeit nicht getroffen werden, während die Wirkstoffe auf die Blätter darunter wachsender unerwünschter Pflanzen oder die unbedeckte Bodenfläche gelangen (post-directed, lay-by).

Die Aufwandmengen an Wirkstoff der Formel I betragen je nach Bekämpfungsziel, Jahreszeit, Zielpflanzen und Wachstumsstadium 0,001 bis 3,0, vorzugsweise 0,01 bis 1,0 kg/ha aktive Substanz (a.S.).

Zur Verbreiterung des Wirkungsspektrums und zur Erzielung synergistischer Effekte können die substituierten Carbamoylderivate der Formel I mit zahlreichen Vertretern anderer herbizider oder wachstumsregulierender Wirkstoffgruppen gemischt und gemeinsam ausgebracht werden. Beispielsweise kommen als Mischungspartner 1,2,4-Thiadiazole, 1,3,4-Thiadiazole, Amide, Aminophosphorsäure und deren Derivate, Aminotriazole, Anilide, Aryloxy-/Heteroaryloxyalkansäuren und deren Derivate, Benzoesäure und deren Derivate, Benzothiadiazinone, 2-(Hetaroyl/Aroyl)-1,3-cyclohexandione, Heteroaryl-Aryl-Ketone, Benzylisoxazolidinone, meta-CF₃-Phenylderivate, Carbamate, Chinolincarbonsäure und deren Derivate, Chloracetanilide, Cyclohexan-1,3-dionderivate, Diazine, Dichlorpropionsäure und deren Derivate, Dihydrobenzofurane, Dihydrofuran-3-one, Dinitroaniline, Dinitrophenole, Diphenylether, Dipyridyle, Halogencarbonsäuren und deren Derivate, Harnstoffe, 3-Phenyluracile, Imidazole, Imidazolinone, N-Phenyl-3,4,5,6-tetrahydrophthalimide, Oxadiazole, Oxirane, Phenole, Aryloxy- und Heteroaryloxyphenoxypropionsäureester, Phenylessigsäure und deren Derivate, 2-Phenylpropionsäure und deren Derivate, Pyrazole, Phenylpyrazole, Pyridazine, Pyridincarbonsäure und deren Derivate, Pyrimidylether, Sulfonamide, Sulfonylharnstoffe, Triazine, Triazinone, Triazolinone, Triazolcarboxamide und Uracile in Betracht.

Außerdem kann es von Nutzen sein, die Verbindungen der Formel I allein oder in Kombination mit anderen Herbiziden auch noch mit weiteren Pflanzenschutzmitteln gemischt, gemeinsam auszubringen, beispielsweise mit Mitteln zur Bekämpfung von Schädlingen oder phytopathogenen Pilzen bzw. Bakterien. Von Interesse ist ferner die Mischbarkeit mit Mineralsalzlösungen, welche zur Behebung von Ernährungs- und Spurenelementmängeln eingesetzt werden. Es können auch nichtphytotoxische Öle und Ölkonzentrate zugesetzt werden.

### Anwendungsbeispiele

Die herbizide Wirkung der substituierten Carbamoylderivate der Formel I ließ sich durch die folgenden Gewächshausversuche zeigen:

Als Kulturgefäße dienten Plastikblumentöpfe mit lehmigem Sand mit etwa 3,0 % Humus als Substrat. Die Samen der Testpflanzen wurden nach Arten getrennt eingesät.

Bei Vorauflaufbehandlung wurden die in Wasser suspendierten oder emulgierten Wirkstoffe direkt nach Einsaat mittels fein verteilender Düsen aufgebracht. Die Gefäße wurden leicht beregnet, um Keimung und Wachstum zu fördern, und anschließend mit durchsichtigen Plastikhauben abgedeckt, bis die Pflanzen angewachsen waren. Diese Abdeckung bewirkt ein gleichmäßiges Keimen der Testpflanzen, sofern dies nicht durch die Wirkstoffe beeinträchtigt wurde. Die Aufwandmenge für die Vorauflaufbehandlung betrug 3,0 kg/ha a.S..

Zum Zweck der Nachauflaufbehandlung wurden die Testpflanzen je nach Wuchsform erst bis zu einer Wuchshöhe von 3 bis 15 cm angezogen und erst dann mit den in Wasser suspendierten oder emulgierten Wirkstoffen behandelt. Die Testpflanzen wurden dafür entweder direkt gesät und in den gleichen Gefäßen aufgezogen oder sie wurden erst als Keimpflanzen getrennt angezogen und einige Tage vor der Behandlung in die Versuchsgefäße verpflanzt.

Die Pflanzen wurden artenspezifisch bei Temperaturen von 10 bis 25°C bzw. 20 bis 35°C gehalten. Die Versuchsperiode erstreckte sich über 2 bis 4 Wochen. Während dieser Zeit wurden die Pflanzen gepflegt, und ihre Reaktion auf die einzelnen Behandlungen wurde ausgewertet.

Bewertet wurde nach einer Skala von 0 bis 100. Dabei bedeutet 100 kein Aufgang der Pflanzen bzw. völlige Zerstörung zumindest der oberirdischen Teile und 0 keine Schädigung oder normaler Wachstumsverlauf.

Die in den Gewächshausversuchen verwendeten Pflanzen setzten sich aus folgenden Arten zusammen:

| Lateinischer Name | Deutscher Name | Englischer Name |
|---|---|---|
| Echinochloa crus-galli | Hühnerhirse | barnyardgrass |
| Lolium multiflorum | Ital. Raygras | annual ryegrass |

Die Verbindungen 2.02 und 2.04 zeigen im Vorauflauf bei Aufwandmengen von 3,0 kg/ha a.S. sehr gute Wirkung gegen die oben genannten Schadgräser.

## Patentansprüche

1. Substituierte Carbamoyltriazole der Formel I 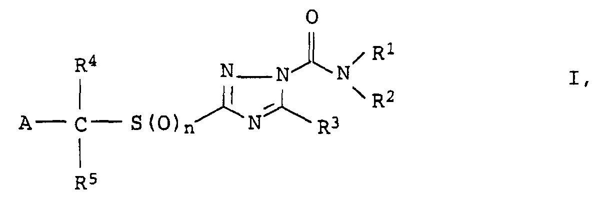 in der die Variablen folgende Bedeutung haben:
R¹ Wasserstoff, C₁-C₆-Alkyl, C₁-C₆-Halogenalkyl, C₃-C₈-Cycloalkyl, C₃-C₆-Alkenyl oder C₃-C₆-Alkinyl;
R² C₁-C₆-Alkoxy oder C₁-C₆-Halogenalkoxy, sowie die unter R¹ genannten Reste;
oder
R¹ und R² bilden gemeinsam einen C₂-C₅-Alkandiyl-Rest, der ein bis drei Substituenten, ausgewählt aus der Gruppe Halogen und C₁-C₆-Alkyl, tragen kann, und im Fall eines C₄-C₅-Alkandiyl-Restes kann eine CH₂-Gruppe durch Sauerstoff oder eine Gruppe NH oder N-C₁-C₆-Alkyl ersetzt werden;
R³,R⁴,R⁵ Wasserstoff, Cyano, C₁-C₆-Alkyl, C₁-C₆-Halogenalkyl, C₃-C₆-Cycloalkyl, C₂-C₆-Alkenyl, C₂-C₆-Alkinyl, C₁-C₆-Alkoxy, C₁-C₆-Halogenalkoxy, C₁-C₆-Alkylthio, C₁-C₆-Halogenalkylthio, C₁-C₆-Alkylcarbonyl, C₁-C₆-Halogenalkylcarbonyl, C₁-C₆-Alkoxycarbonyl, Phenyl oder Benzyl, wobei die beiden letztgenannten Substituenten partiell oder vollständig halogeniert sein können und ein bis drei der folgenden Gruppen tragen können:
Nitro, Cyano, C₁-C₄-Alkyl, C₁-C₄-Halogenalkyl, C₁-C₄-Alkoxy, C₁-C₄-Halogenalkoxy und C₁-C₄-Alkoxycarbonyl;
A ggf. substituierter, gesättigter oder partiell ungesättigter Heterocyclus, der ein bis drei Heteroatome, ausgewählt aus der Gruppe Sauerstoff, Schwefel und Stickstoff enthält, und fünf- oder sechsgliedrig ist;
n 0, 1 oder 2;
mit der Maßgabe, daß A nicht für 1,3-Dioxolan-2-yl oder 1,3-Dioxan-2-yl steht;
sowie die landwirtschaftlich brauchbaren Salze von I.

2. Substituierte Carbamoyltriazole der Formel I gemäß Anspruch 1, in der
A fünfgliedriger, gesättigter oder partiell ungesättigter Heterocyclus, der ein bis drei Heteroatome, ausgewählt aus der Gruppe Sauerstoff, Schwefel und Stickstoff, enthält und ein bis drei an Kohlenstoff gebundene Reste, ausgewählt aus der Gruppe R⁶, R⁷ und R⁸, tragen kann und wobei ggf. jeder Ringstickstoff unabhängig voneinander einen Rest R¹⁰ tragen kann;
oder
sechsgliedriger, gesättigter oder partiell ungesättigter Heterocyclus, der ein bis drei Heteroatome, ausgewählt aus der Gruppe Sauerstoff, Schwefel und Stickstoff, enthält und ein bis vier an Kohlenstoff gebundene Reste, ausgewählt aus der Gruppe R⁶, R⁷, R⁸ und R⁹, tragen kann und wobei ggf. jeder Ringstickstoff unabhängig voneinander einen Rest R¹⁰ tragen kann;
R⁶,R⁷,R⁸,R⁹ Cyano, Halogen, C₁-C₆-Alkyl, C₁-C₆-Halogenalkyl, C₃-C₆-Cycloalkyl, C₂-C₆-Alkenyl, C₂-C₆-Alkinyl, C₁-C₆-Alkoxy, C₁-C₆-Halogenalkoxy, C₁-C₆-Alkylthio, C₁-C₆-Halogenalkylthio, C₁-C₆-Alkylcarbonyl, C₁-C₆-Halogenalkylcarbonyl, C₁-C₆-Alkoxycarbonyl, Phenyl oder Benzyl, wobei die beiden letztgenannten Substituenten partiell oder vollständig halogeniert sein können und/oder eine bis drei der folgenden Gruppen tragen können:
Nitro, Cyano, C₁-C₄-Alkyl, C₁-C₄-Halogenalkyl, C₁-C₄-Alkoxy, C₁-C₄-Halogenalkoxy und C₁-C₄-Alkoxycarbonyl;
R¹⁰ C₁-C₆-Alkyl, C₁-C₆-Halogenalkyl, C₃-C₆-Cycloalkyl, C₃-C₆-Alkenyl, C₃-C₆-Alkinyl, C₁-C₆-Alkylcarbonyl, C₁-C₆-Halogenalkylcarbonyl, C₁-C₆-Alkoxycarbonyl, Phenyl oder Benzyl, wobei die beiden letztgenannten Substituenten partiell oder vollständig halogeniert sein können und/oder eine bis drei der folgenden Gruppen tragen können: Nitro, Cyano, C₁-C₄-Alkyl, C₁-C₄-Halogenalkyl, C₁-C₄-Alkoxy, C₁-C₄-Halogenalkoxy und C₁-C₄-Alkoxycarbonyl;
bedeutet.

3. Substituierte Carbamoyltriazole der Formel I, nach den Ansprüchen 1 oder 2, in der
R¹, R² für C₁-C₆-Alkyl stehen.

4. Substituierte Carbamoyltriazole der Formel I, nach den Ansprüchen 1 bis 3, in der
R³, R⁴ für Wasserstoff stehen.

5. Verfahren zur Herstellung von substituierten Carbamoyltriazolen der Formel I, gemäß den Ansprüchen 1 bis 4, **dadurch gekennzeichnet, daß** man ein Triazol der Formel II 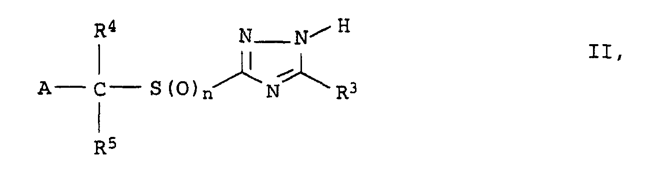 mit einem Carbamidsäurehalogenid der Formel III 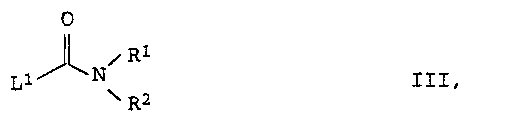 umsetzt, wobei R¹ bis R⁵, A und n die in den Ansprüchen 1 bis 4 angegebene Bedeutung haben und L¹ für Halogen steht.

6. Verfahren zur Herstellung von substituierten Carbamoyltriazolen der Formel I mit n = 1 oder 2, gemäß den Ansprüchen 1 bis 4, **dadurch gekennzeichnet, daß** man ein substituiertes Carbamoyltriazol der Formel I mit n = 0 gemäß den Ansprüchen 1 bis 4 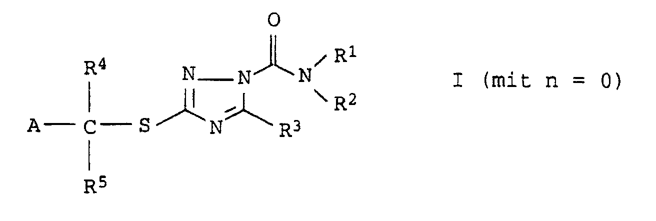 mit einem Oxidationsmittel behandelt, wobei R¹ bis R⁵ und A die in den Ansprüchen 1 bis 4 angegebene Bedeutung haben.

7. Triazole der Formel II 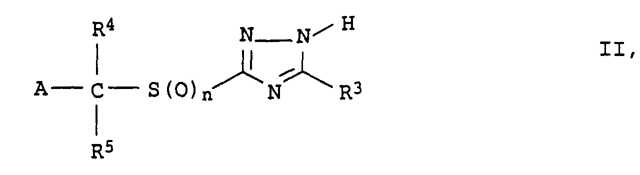 wobei die Variablen R³ bis R⁵, A und n folgende Bedeutung haben:
R³,R⁴,R⁵ Wasserstoff, Cyano, C₁-C₆-Alkyl, C₁-C₆-Halogenalkyl, C₃-C₆-Cycloalkyl, C₂-C₆-Alkenyl, C₂-C₆-Alkinyl, C₁-C₆-Alkoxy, C₁-C₆-Halogenalkoxy, C₁-C₆-Alkylthio, C₁-C₆-Halogenalkylthio, C₁-C₆-Alkylcarbonyl, C₁-C₆-Halogenalkylcarbonyl, C₁-C₆-Alkoxycarbonyl, Phenyl oder Benzyl, wobei die beiden letztgenannten Substituenten partiell oder vollständig halogeniert sein können und eine bis drei der folgenden Gruppen tragen können:
Nitro, Cyano, C₁-C₄-Alkyl, C₁-C₄-Halogenalkyl, C₁-C₄-Alkoxy, C₁-C₄-Halogenalkoxy und C₁-C₄-Alkoxycarbonyl;
A ggf. substituierter, gesättigter oder partiell ungesättigter Heterocyclus, der ein bis drei Heteroatome, ausgewählt aus der Gruppe Sauerstoff, Schwefel und Stickstoff enthält, und fünf- oder sechsgliedrig ist;
n 2;
mit der Maßgabe, daß A nicht für 1,3-Dioxolan-2-yl oder 1,3-Dioxan-2-yl steht.

8. Herbizides Mittel, enthaltend eine herbizid wirksame Menge mindestens eines substituierten Carbamoyltriazols der Formel I gemäß den Ansprüchen 1 bis 4 oder eines landwirtschaftlich brauchbaren Salzes desselben, und mindestens einen inerten flüssigen und/oder festen Trägerstoff sowie gewünschtenfalls mindestens einen oberflächenaktiven Stoff.

9. Verfahren zur Herstellung von herbizid wirksamen Mitteln gemäß Anspruch 8, **dadurch gekennzeichnet, daß** man eine herbizid wirksame Menge mindestens eines substituierten Carbamoyltriazols der Formel I gemäß den Ansprüchen 1 bis 4 oder eines landwirtschaftlich brauchbaren Salzes desselben, und mindestens einen inerten flüssigen und/oder festen Trägerstoff sowie gewünschtenfalls mindestens einen oberflächenaktiven Stoff mischt.

10. Verfahren zur Bekämpfung von unerwünschtem Pflanzenwuchs, **dadurch gekennzeichnet, daß** man eine herbizid wirksame Menge mindestens eines substituierten Carbamoyltriazols der Formel I gemäß den Ansprüchen 1 bis 4 oder eines landwirtschaftlich brauchbaren Salzes desselben, auf Pflanzen, deren Lebensraum oder auf Samen einwirken läßt.

11. Verwendung der substituierten Carbamoyltriazole der Formel I gemäß den Ansprüchen 1 bis 4 oder deren landwirtschaftlich brauchbaren Salze als Herbizide.

## Claims

1. Substituted carbamoyltriazoles of the formula I 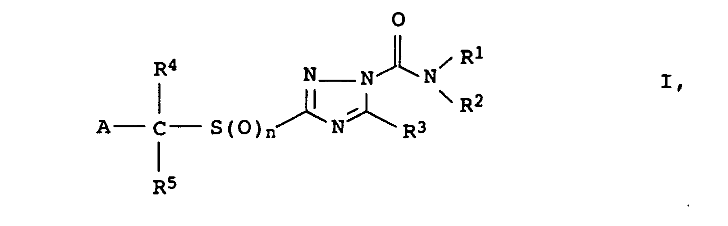 where
R¹ is hydrogen, C₁-C₆-alkyl, C₁-C₆-haloalkyl, C₃-C₈-cycloalkyl, C₃-C₆-alkenyl or C₃-C₆-alkynyl;
R² is C₁-C₆-alkoxy or C₁-C₆-haloalkoxy or a radical listed under R¹;
or
R¹ and R² together form a C₂-C₅-alkanediyl radical which may carry one to three substituents selected from the group consisting of halogen and C₁-C₆-alkyl and, in the case of a C₄-C₅-alkanediyl radical, a CH₂ group may be replaced by oxygen or a group NH or N-C₁-C₆-alkyl;
R³,R⁴ and R⁵ are each hydrogen, cyano, C₁-C₆-alkyl, C₁-C₆-haloalkyl, C₃-C₆-cycloalkyl, C₂-C₆-alkenyl, C₂-C₆-alkynyl, C₁-C₆-alkoxy, C₁-C₆-haloalkoxy, C₁-C₆-alkylthio, C₁-C₆-haloalkylthio, C₁-C₆-alkylcarbonyl, C₁-C₆-haloalkylcarbonyl, C₁-C₆-alkoxycarbonyl, phenyl or benzyl, where the last two substituents may be partially or fully halogenated and may carry one to three of the following groups:
nitro, cyano, C₁-C₄-alkyl, C₁-C₄-haloalkyl, C₁-C₄-alkoxy, C₁-C₄-haloalkoxy and C₁-C₄-alkoxycarbonyl;
A is an unsubstituted or substituted saturated or partially unsaturated five- or six-membered heterocycle containing one to three hetero atoms selected from the group consisting of oxygen, sulfur and nitrogen;
n is 0, 1 or 2;
with the proviso that A is not 1,3-dioxolan-2-yl or 1,3-dioxan-2-yl;
and the agriculturally useful salts of I.

2. Substituted carbamoyltriazoles of the formula I as claimed in claim 1, where
A is a five-membered saturated or partially unsaturated heterocycle which contains one to three hetero atoms selected from the group consisting of oxygen, sulfur and nitrogen and which may carry, attached to carbon, one to three radicals selected from the group consisting of R⁶, R⁷ and R⁸, where any ring nitrogen may, independently of any other(s), carry a radical R¹⁰;
or
is a six-membered saturated or partially unsaturated heterocycle which contains one to three hetero atoms selected from the group consisting of oxygen, sulfur and nitrogen and which may carry, attached to carbon, one to four radicals selected from the group consisting of R⁶, R⁷, R⁸ and R⁹, where any ring nitrogen may, independently of any other(s), carry a radical R¹⁰;
R⁶,R⁷,R⁸ and R⁹ are each cyano, halogen, C₁-C₆-alkyl, C₁-C₆-haloalkyl, C₃-C₆-cycloalkyl, C₂-C₆-alkenyl, C₂-C₆-alkynyl, C₁-C₆-alkoxy, C₁-C₆-haloalkoxy, C₁-C₆-alkylthio, C₁-C₆-haloalkylthio, C₁-C₆-alkylcarbonyl, C₁-C₆-haloalkylcarbonyl, C₁-C₆-alkoxycarbonyl, phenyl or benzyl where the last two substituents may be partially or fully halogenated and/or may carry one to three of the following groups:
nitro, cyano, C₁-C₄-alkyl, C₁-C₄-haloalkyl, C₁-C₄-alkoxy, C₁-C₄-haloalkoxy and C₁-C₄-alkoxycarbonyl;
R¹⁰ is C₁-C₆-alkyl, C₁-C₆-haloalkyl, C₃-C₆-cycloalkyl, C₃-C₆-alkenyl, C₃-C₆-alkynyl, C₁-C₆-alkylcarbonyl, C₁-C₆-haloalkylcarbonyl, C₁-C₆-alkoxycarbonyl, phenyl or benzyl where the last two substituents may be partially or fully halogenated and/or may carry one to three of the following groups:
nitro, cyano, C₁-C₄-alkyl, C₁-C₄-haloalkyl, C₁-C₄-alkoxy, C₁-C₄-haloalkoxy and C₁-C₄-alkoxycarbonyl.

3. Substituted carbamoyltriazoles of the formula I as claimed in claim 1 or 2 where
R¹ and R² are each C₁-C₆-alkyl.

4. Substituted carbamoyltriazoles of the formula I as claimed in any of claims 1 to 3 where
R³ and R⁴ are each hydrogen.

5. A process for preparing substituted carbamoyltriazoles of the formula I as claimed in any of claims 1 to 4, which comprises reacting a triazole of the formula II 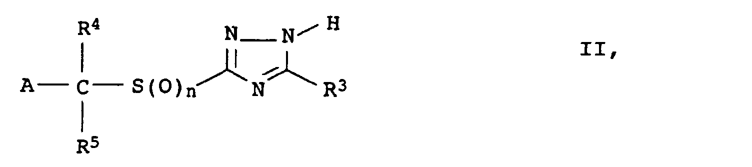 with a carbamoyl halide of the formula III 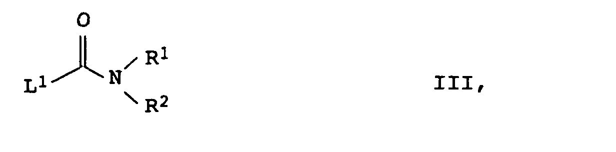 where R¹ to R⁵, A and n are each as defined in any of claims 1 to 4 and L¹ is halogen.

6. A process for preparing substituted carbamoyltriazoles of the formula I where n = 1 or 2 as claimed in any of claims 1 to 4, wherein a substituted carbamoyltriazole of the formula I where n = 0 as claimed in any of claims 1 to 4 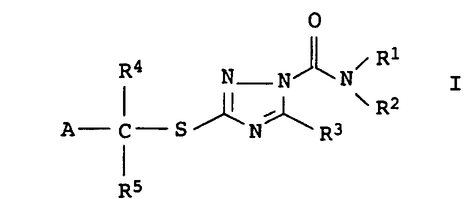 (where n = 0)
is treated with an oxidizing agent where R¹ to R⁵ and A each are as defined in any of claims 1 to 4.

7. Triazoles of the formula II 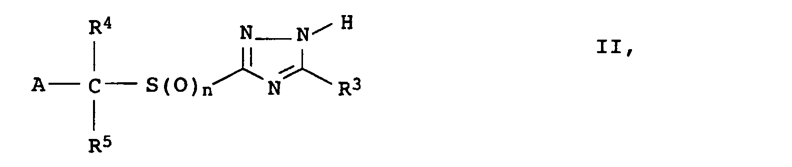 where
R³,R⁴ and R⁵ are each hydrogen, cyano, C₁-C₆-alkyl, C₁-C₆-haloalkyl, C₃-C₆-cycloalkyl, C₂-C₆-alkenyl, C₂-C₆-alknyl, C₁-C₆-alkoxy, C₁-C₆-haloalkoxy, C₁-C₆-alkylthio, C₁-C₆-haloalkylthio, C₁-C₆-alkylcarbonyl, C₁-C₆-haloalkylcarbonyl, C₁-C₆-alkoxycarbonyl, phenyl or benzyl, where the last two substituents may be partially or fully halogenated and may carry one to three of the following groups:
nitro, cyano, C₁-C₄-alkyl, C₁-C₄-haloalkyl, C₁-C₄-alkoxy, C₁-C₄-haloalkoxy and C₁-C₄-alkoxycarbonyl;
A is an unsubstituted or substituted saturated or partially unsaturated five- or six-membered heterocycle containing one to three hetero atoms selected from the group consisting of oxygen, sulfur and nitrogen;
n is 2;
with the proviso that A is not 1,3-dioxolan-2-yl or 1,3-dioxan-2-yl.

8. A herbicidal composition comprising a herbicidally active amount of at least one substituted carbamoyltriazole of the formula I as claimed in any of claims 1 to 4 or of an agriculturally useful salt thereof and at least one inert liquid and/or solid carrier and, if desired, at leasst one surfactant.

9. A process for preparing herbicidally active compositions as claimed in claim 8, which comprises mixing a herbicidally active amount of at least one substituted carbamoyltriazole of the formula I as claimed in any of claims 1 to 4 or of an agriculturally useful salt thereof and at least one inert liquid and/or solid carrier and, if desired, at least one surfactant.

10. A method of controllling undesirable vegetation, which comprises allowing a herbicidally active amount of at least one substituted carbamoyltriazole of the formula I as claimed in any of claims 1 to 4 or of an agriculturally useful salt thereof to act on plants, their habitat or on seeds.

11. The use of the substituted carbamoyltriazoles of the formula I as claimed in any of claims 1 to 4 or their agriculturally useful salts as herbicides.

## Revendications

1. Carbamoyltriazoles substitués de formule I 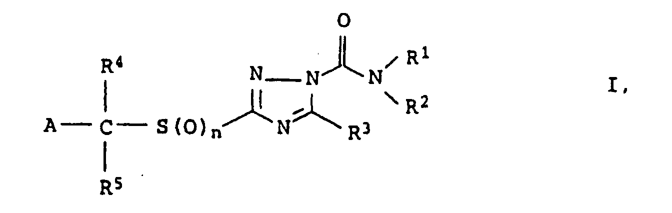 dans laquelle les symboles ont les significations suivantes :
R¹ : l'hydrogène, un groupe alkyle en C1-C6, halogénoalkyle en Cl-C6, cycloalkyle en C3-C8, alcényle en C3-C6 ou alcynyle en C3-C6 ;
R² : un groupe alcoxy en C1-C6 ou halogénoalcoxy en C1-C6 ou l'un des groupes mentionnés en référence à R¹ ;
ou bien
R¹ et R² forment ensemble un groupe alcanediyle en C2-C5 qui peut porter un à trois substituants choisis parmi les halogènes et les groupes alkyle en C1-C6, et dans le cas d'un groupe alcanediyle en C4-C5, un groupe CH₂ peut être remplacé par l'oxygène ou par un groupe NH ou N-alkyle en C1-C2 ;
R³, R⁴, R⁵: l'hydrogène, des groupes cyano, alkyle en C1-C6, halogénoalkyle en C1-C6, cycloalkyle en C3-C6, halogénoalkyle en C1-C6, cycloalkyle en C3-C6, alcényle en C2-C6, alcynyle en C2-C6, alcoxy en C1-C6, halogénoalcoxy en C1-C6, alkylthio en C1-C6, halogénoalkylthio en C1-C6, (alkyle en C1-C6)carbonyle, (halogénoalkyle en C1-C6)carbonyle, (alcoxy en C1-C6)carbonyle, phényle ou benzyle, ces deux derniers substituants pouvant être halogénés en totalité ou en partie et pouvant porter un à trois des groupes suivants :
nitro, cyano, alkyle en C1-C4, halogénoalkyle en C1-C4, alcoxy en Cl-C4, halogénoalcoxy en C1-C4 et (alcoxy en C1-C4)carbonyle ;
A : un hétérocycle saturé ou partiellement insaturé, éventuellement substitué, qui contient un à trois hétéroatomes choisis parmi l'oxygène, le soufre et l'azote, et possède cinq ou six chaînons ;
n : 0, 1 ou 2 ;
sous réserve que A ne peut représenter un groupe 1,3-dioxolan-2-yle ou 1,3-dioxan-2-yle ;
et les sels de I aptes aux applications agricoles.

2. Carbamoyltriazoles substitués de formule I selon la revendication 1 dans laquelle
A représente un hétérocycle saturé ou partiellement insaturé à cinq chaînons qui contient un à trois hétéroatomes choisis parmi l'oxygène, le soufre et l'azote et peut porter un à trois groupes fixés sur le carbone, choisis parmi les groupes R⁶, R⁷ et R⁸, chaque azote cyclique pouvant éventuellement porter, indépendamment des autres, un groupe R¹⁰ ;
ou bien
un hétérocycle saturé ou partiellement insaturé à six chaînons qui contient un à trois hétéroatomes choisis parmi l'oxygène, le soufre et l'azote et peut porter un à quatre groupes fixés sur le carbone choisis parmi les groupes R⁶, R⁷, R⁸ et R⁹, chaque azote cyclique pouvant le cas échéant porter, indépendamment des autres, un groupe R¹⁰ ;
R⁶, R⁷, R⁸, R⁹ : des groupes cyano, des halogènes, des groupes alkyle en C1-C6, halogénoalkyle en C1-C6, cycloalkyle en C3-C6, alcényle en C2-C6, alcynyle en C2-C6, alcoxy en C1-C6, halogénoalcoxy en C1-C6, alkylthio en C1-C6, halogénoalkylthio en C1-C6, (alkyle en C1-C6)carbonyle, (halogénoalkyle en C1-C6)carbonyle, (alcoxy en C1-C6)carbonyle, phényle ou benzyle, ces deux derniers substituants pouvant être halogénés en totalité ou en partie et/ou pouvant porter un à trois des groupes suivants :
nitro, cyano, alkyle en C1-C4, halogénoalkyle en C1-C4, alcoxy en C1-C4, halogénoalcoxy en C1-C4 et (alcoxy en C1-C4)carbonyle ;
R¹⁰ : un groupe alkyle en C1-C6, halogénoalkyle en C1-C6, cycloalkyle en C3-C6, alcényle en C3-C6, alcynyle en C3-C6, (alkyle en C1-C6)carbonyle, (halogénoalkyle en C1-C6)carbonyle, (alcoxy en C1-C6)carbonyle, phényle ou benzyle, ces deux derniers substituants pouvant être halogénés en totalité ou en partie et/ou pouvant porter un à trois des groupes suivants : nitro, cyano, alkyle en C1-C4, halogénoalkyle en C1-C4, alcoxy en C1-C4, halogénoalcoxy en C1-C4 et (alcoxy en C1-C4)carbonyle.

3. Carbamoyltriazoles substitués de formule I selon la revendication 1 ou 2, dans laquelle
R¹, R² représentent des groupes alkyle en C1-C6.

4. Carbamoyltriazoles substitués de formule I selon l'une des revendications 1 à 3, dans laquelle
R³, R⁴ représentent l'hydrogène.

5. Procédé pour la préparation des carbamoyltriazoles substitués de formule I selon les revendications 1 à 4, **caractérisé par le fait que** l'on fait réagir un triazole de formule II 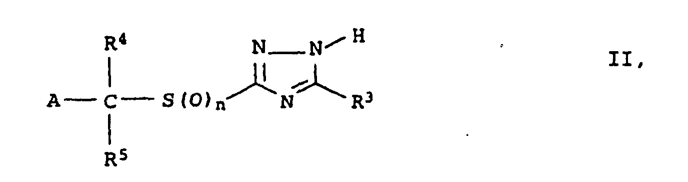 avec un halogénure d'acide carbamique de formule III 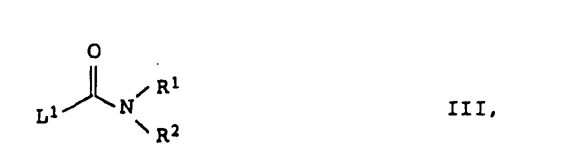 dans lesquelles R¹ à R⁵, A et n ont les significations indiquées dans les revendications 1 à 4 et L¹ représente un halogène.

6. Procédé pour la préparation des carbamoyltriazoles substitués de formule I dans laquelle n = 1 ou 2 selon les revendications 1 à 4, **caractérisé par le fait que** l'on traite un carbamoyltriazole substitué de formule I dans laquelle n = 0 selon les revendications 1 à 4 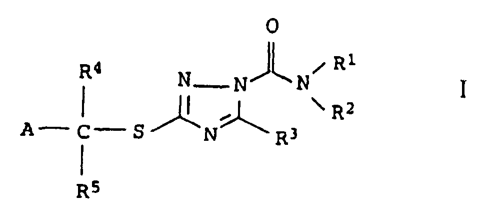 (avec n = 0)
par un agent oxydant, R¹ à R⁵ et A ayant les significations indiquées dans les revendications 1 à 4.

7. Triazoles de formule II 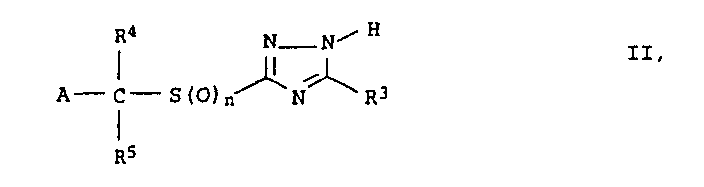 dans laquelle les symboles R³ à R⁵, A et n ont les significations suivantes:
R³, R⁴, R⁵: l'hydrogène, des groupes cyano, alkyle en C1-C6, halogénoalkyle en C1-C6, cycloalkyle en C3-C6, alcényle en C2-C6, alcynyle en C2-C6, alcoxy en C1-C6, halogénoalcoxy en C1-C6, alkylthio en C1-C6, halogénoalkylthio en C1-C6, (alkyle en Cl-C6)carbonyle, (halogénoalkyle en C1-C6)carbonyle, (alcoxy en Cl-C6)carbonyle, phényle ou benzyle, ces deux derniers substituants pouvant être halogénés en totalité ou en partie et pouvant porter un à trois des groupes suivants : nitro, cyano, alkyle en C1-C4, halogénoalkyle en C1-C4, alcoxy en C1-C4, halogénoalcoxy en C1-C4 et (alcoxy en C1-C4)carbonyle ;
A : un hétérocycle saturé ou partiellement insaturé, éventuellement substitué, qui contient un à trois hétéroatomes choisis parmi l'oxygène, le soufre et l'azote et possède cinq ou six chaînons ;
n:2;
sous réserve que A ne peut représenter un groupe 1,3-dioxolan-2-yle ou 1,3-dioxan-2-yle.

8. Produit herbicide contenant une quantité herbicide efficace d'au moins un carbamoyltriazole substitué de formule I selon les revendications 1 à 4 ou de l'un de ses sels aptes aux applications agricoles, et au moins un véhicule liquide et/ou solide inerte ainsi que, si on le désire, au moins une substance tensio-active.

9. Procédé pour la préparation de produits à activité herbicide selon la revendication 8, **caractérisé par le fait que** l'on mélange une quantité herbicide efficace d'au moins un carbamoyltriazole substitué de formule I selon les revendications 1 à 4 ou de l'un de ses sels aptes aux applications agricoles, et au moins un véhicule liquide et/ou solide inerte ainsi que, si on le désire, au moins une substance tensio-active.

10. Procédé pour combattre les croissances de végétaux indésirables, **caractérisé par le fait que** l'on fait agir une quantité herbicide efficace d'au moins un carbamoyltriazole substitué de formule I selon les revendications 1 à 4 ou de l'un de ses sels aptes aux applications agricoles sur les végétaux, leur habitat ou sur les semences.

11. Utilisation des carbamoyltriazoles substitués de formule I selon les revendications 1 à 4 ou de leurs sels aptes aux applications agricoles en tant qu'herbicides.
